(19) Europäisches Patentamt
European Patent Office
Office européen des brevets

(11) EP 4 206 202 A1

(12) EUROPEAN PATENT APPLICATION

(43) Date of publication:
**05.07.2023 Bulletin 2023/27**

(21) Application number: **21306966.9**

(22) Date of filing: **31.12.2021**

(51) International Patent Classification (IPC):
***C07D 471/08*** *(2006.01)* ***C07B 59/00*** *(2006.01)*

(52) Cooperative Patent Classification (CPC):
**C07D 471/08**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Université de Strasbourg
67000 Strasbourg (FR)**
- **Centre national de la recherche scientifique
75016 Paris (FR)**

(72) Inventors:
- **CHARBONNIERE, Loïc
67720 Weyersheim (FR)**
- **SY, Maryame
94600 CHOISY-LE-ROI (FR)**
- **Jakab Tóth, Eva
45160 OLIVET (FR)**
- **NONAT, Aline
67201 ECKBOLSHEIM (FR)**

(74) Representative: **IPAZ
Bâtiment Platon
Parc Les Algorithmes
91190 Saint-Aubin (FR)**

# (54) NOVEL BISPIDINE-BASED METAL CHELATING LIGANDS DISPLAYING GOOD RELAXIVITY

(57) The present invention relates to novel bispidine-based compounds comprising at least an optionally substituted ethanoic acid moiety at the N7-position of the bispidine scaffold, said bispidine-based compounds representing metal chelating ligands able to form metallic complexes having good properties in terms of relaxivity and stability, to complexes comprising a metal ion complexed with said bispidine-based compounds, and to the use of said bispidine-based compounds in the field of medical imaging or therapy, and more specifically MRI (magnetic resonance imaging) contrast agents and/or nuclear imaging agents for PET (positron emission tomography) and/or SPECT (single photon emission tomography).

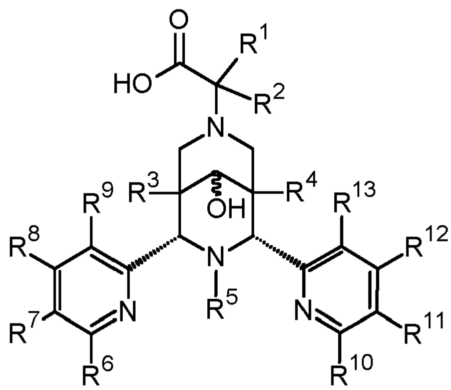

**(I)**

**FIG. 1**



Processed by Luminess, 75001 PARIS (FR)

## Description

The present invention relates to novel bispidine-based compounds comprising at least an optionally substituted ethanoic acid moiety at the N7-position of the bispidine scaffold, said bispidine-based compounds representing metal chelating ligands able to form metallic complexes having good properties in terms of relaxivity and stability, to complexes comprising a metal ion complexed with said bispidine-based compounds, and to the use of said bispidine-based compounds in the field of medical imaging or therapy, and more specifically as MRI (magnetic resonance imaging) contrast agents and/or nuclear imaging agents for PET (positron emission tomography) or SPECT (single photon emission tomography).

Gadolinium-complexes have been used in millions of human examinations with magnetic resonance imaging (MRI) and are considered among the safest diagnostic drugs. The following gadolinium-based contrast agents (GBCAs) have been approved for clinical use: gadopentetate dimeglumine or $[Gd(OH_2)(dtpa)]^{2-}$ (Magnevist®, compound "a" in Scheme 1 below), gadoteridol (ProHance®), gadodiamide or $[Gd(OH_2)(dtpa\text{-}bma)]$ (Omniscan®, compound "b" in Scheme 1 below), gadoterate meglumine or $[Gd(OH_2)(dota)]^-$ (Dotarem®, compound "c" in Scheme 1 below), gadobutrol or $[Gd(OH_2)(do3a\text{-}butrol)]$ (Gadovist®, compound "d" in Scheme 1 below), gadoversetamide (OptiMARK®), gadoxetic acid (Primovist®), gadobenate dimeglumine or $[Gd(OH_2)(bopta)]^{2-}$ (MultiHance®, compound e in Scheme 1 below), and gadofosveset trisodium (Vasovist®/Ablavar®).

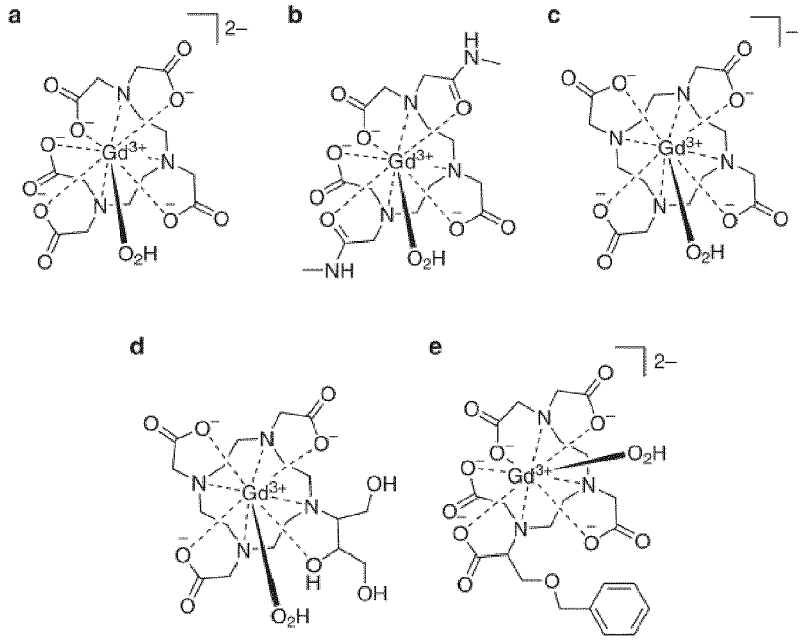

a b c d e

**SCHEME 1**

However, the recent emergence of nephrogenic systemic fibrosis and its causal link to Gd exposure, as well as the evidence on brain and bone accumulation of Gd have alerted the medical community. Nephrogenic systemic fibrosis (NSF) is a rare and serious syndrome that is associated with the exposure to GBCAs in patients with chronic kidney disease. NSF involves fibrotic changes in the skin and many organs.

Therefore, in 2010, the U.S. Food and Drug Administration (FDA) published revised labeling recommendations for four linear GBCAs which have been principally implicated in NSF, including gadodiamide (Omniscan®), gadobenate dimeglumine (MultiHance®), gadopentetate dimeglumine (Magnevist®), and gadoversetamide (OptiMARK®). In 2017, the European Medicines Agency has decided to stop marketing linear GBCAs contrast agents.

Furthermore, the negative outcome of using high quantities of Gd(III) based contrast agents, e.g. gadolinium accumulating in surface waters and coming from clinical waste waters, also arises an increasing environmental problem.

In this context, providing novel Gd(III) based contrast agents that can be administered in lower quantities and/or that are more stable as well as replacing Gd(III) with more biocompatible, safer paramagnetic metal ions have become major objectives.

Indeed, due to the low sensitivity of MRI as an imaging technique, large quantities of a contrast agent, often on the gram scale, must be injected into the patient to obtain useful images. The ability to reduce the quantity of GBCAs required is highly desirable, especially when considering the toxicity problems discussed above. One way in which the

amount of contrast agent required can be reduced is to enhance its relaxivity. Relaxivity is the ability of the metal chelate to relax water protons, and is defined as the change in the relaxation rate of water divided by the millimolar concentration of the chelate. High relaxivities are indicative of more effective agents. All the commercially available GBCAs are very similar to each other in terms of relaxivity. As an example, Dotarem® displays a relaxivity $r_1$ of 3.7 $mM^{-1}.s^{-1}$ at pH of 7.4, 60 MHz, and 25°C.

Another way to reduce or avoid the risks of health problems associated with $Gd^{3+}$ release is to provide stable GBCAs. $Gd^{3+}$ release by GBCAs can be characterised by thermodynamic stability and kinetic inertness. Thermodynamic stability refers to the Gibbs free energy involved in the complexation reaction and is defined by the stability constant log $K$ (also called log $K_{GdL}$, log $K_{therm}$ and log $K_{st}$). Kinetic inertness refers to complex dissociation rate and is mainly reported as $t_{1/2}$, where $t_{1/2}$ is defined as the time required for half of the GBCA's dissociation.

Research is also focused on providing alternative gadolinium(III)-free metal complexes. For example, US2020/157099 A1 describes Mn(II), Fe(II), Fe(III), Co(II) and Ni(II) ion macrocycle-based complexes to apply as MRI contrast agents and $^{52}Mn$-based PET diagnostic. The compound responding to the following formula:

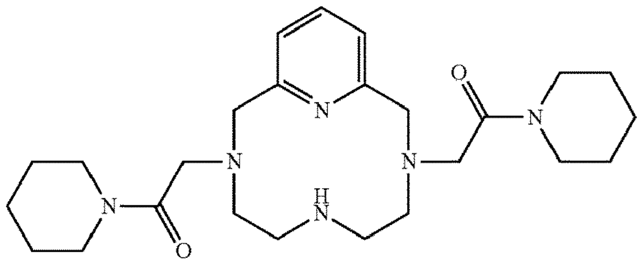

is complexed with Mn(II) so as to form a Mn(III) macrocycle metal complexe [Mn(tPC2AM$^{Pyp}$)]$^{2+}$ having a relaxivity $r_1$ of 4.90 $mM^{-1}.s^{-1}$ at pH of 7.4 and 25°C and an half-life $t_{1/2}$ of complex dissociation calculated at physiologic pH, of 352 hours. However, the relaxivity as well as kinetic interness are not completely satisfactory.

Mangafodipir trisodium (Mn-DPDP), a contrast agent free of Gd(III), was marketed in the 1990's under the tradename Teslascan® comprising Mn(II) ion as the central paramagnetic ion. However, it was withdrawn from the US market in 2003 and from the EU market in 2010 due to low sales, poor clinical performance, and concerns over toxicity.

Accordingly, there is a need of novel metal chelating ligands able to form complexes with other metals than gadolinium(III) and which have improved relaxivity while guaranteeing good kinetic inertness.

Thus, the aim of the present invention is to overcome the drawbacks of the cited prior art, and more particularly, to provide novel metal chelating ligands having good relaxation properties, low toxicity and/or which can be easily eliminated by renal route, water solubility and improved kinetic nertness and/or low dissociation rate.

A first object of the present invention is a bispidine-based metal chelating ligand responding to the following formula (I):

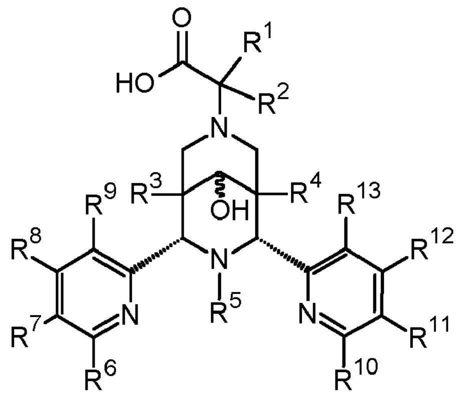

**(I)**

in which:

* $R^1$ represents an hydrogen atom or a $C_1$-$C_5$ alkyl group,

* $R^2$ represents an hydrogen atom, a $C_1$-$C_5$ alkyl group, a group responding to formula (II): $-(CH_2)_n-NH_2$ (II), where

$3 \leq n \leq 18$, or a group responding to the following formula (III):

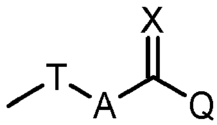

**(III)**

where:

* T represents a $C_1$-$C_{17}$ alkylene group, a $C_1$-$C_{17}$ alkenylene group, or a $C_1$-$C_{17}$ alkynylene group,

* A represents -CH2- or -NH-,

* X represents an oxygen atom, a sulfur atom, or a NH group, and

* Q represents:

- an alkyl group optionally substituted with a functional group,
- a $NHR^{14}$ group where $R^{14}$ represents an hydrogen atom, or an aryl group optionally substituted with a functional group,
- a polyethylene glycol group responding to formula (IV): $-CH_2-(CH_2-O-CH_2)_q-CH_2OH$ (IV), where $1 \leq q \leq 24$, or
- an OH group,

* $R^3$ and $R^4$, which may be identical or different, and preferably identical, represent a $CH_2OH$ group, a $CO_2H$ group, or a $CONHR^{15}$ group, where $R^{15}$ represents:

- an alkyl group optionally substituted with a functional group,
- an aryl group optionally substituted with a functional group, or
- a polyethylene glycol group responding to formula (V): $-CH_2-(CH_2-O-CH_2)_r-CH_2OH$ (V), where $1 \leq r \leq 24$,

* $R^5$ represents an hydrogen atom, an alkyl group, a group of formula (VI): $-(CH_2)_m-CO_2H$ (VI) where $1 \leq m \leq 5$, a polyethylene glycol group responding to formula (VII): $-CH_2-(CH_2-O-CH_2)_s-CH_2OH$ (VII), where $1 \leq s \leq 18$, or a group of formula (III) as defined above,

* $R^6$, $R^7$, $R^8$, $R^9$, $R^{10}$, $R^{11}$, $R^{12}$, and $R^{13}$, which may be identical or different, represent a hydrogen atom, an OH group, an ether group $OR^{16}$ where $R^{16}$ is an alkyl group, a $CO_2H$ group, or a $CONHR^{17}$ group, where $R^{17}$ represents an alkyl group optionally substituted with a functional group,
with the proviso that:

- when $R^2$ represents a group responding to formula (II): $-(CH_2)_n-NH_2$ (II), and $R^5$ represents an alkyl group, then $R^3$ and $R^4$ are different from $CO_2H$ groups,
- when $R^5$ represents an alkyl group, then $R^1$ and $R^2$ are different from hydrogen atoms, and
- when $R^2$ represents a group of said formula (III), where T represents a $C_1$-$C_{17}$ alkylene group, A represents -NH-, X represents an oxygen atom, and Q represents a $C_1$-$C_5$ alkyl group substituted with a functional group, then $R^3$ and $R^4$ are different from $CO_2H$ groups.

Thus, the ligands of the invention can lead to metal complexes having good relaxation properties, low toxicity and/or which can be easily eliminated by renal route, water solubility and improved kinetic nertness and/or low dissociation

rate.

Bispidines are chelators based on a 3,7-diazabicyclo[3.3.1]nonane scaffold. The bispidine scaffold is formed by two fused cyclohexylamine rings.

More particularly, the metal chelating ligand (I) of the present invention has a so-called 2,4-disubstituted bispidol core and its IUPAC numbering is presented as follows:

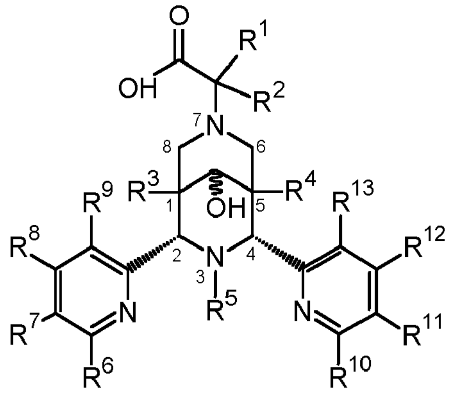

**(I)**

Said metal chelating ligand (I) is expected to coordinate at least in a fivedentate manner, involving two pyridine and two bispidine nitrogens as well as the methylene carboxylate at the N7 position and appropriate $R^1$ and $R^2$ groups on the methylene moiety.

The bispidine-based metal chelating ligands of the present invention are highly preorganized ligands that can accommodate metal ions with cis-octahedral, square-pyramidal, or pentagonal geometries. The bispidine-based metal chelating ligands of the invention form thermodynamically very stable metal complexes with transition-metal ions which show high kinetic inertness. Modification of the coordinating pendant arms can be used to tune the ligand denticity as well as all electronic, thermodynamic, and kinetic parameters such as the ligand field, the metal selectivity, and the stability constants. Such properties are very appealing for applications in diagnosis as chelators for metals other than gadolinium, and more specifically for manganese and copper metals, as well as radiometals such as $^{64}Cu$.

<u>The $R^1$ group</u>

The $R^1$ group represents an hydrogen atom or a $C_1$-$C_5$ alkyl group.

The $C_1$-$C_5$ alkyl group as the $R^1$ group is preferably a linear $C_1$-$C_5$ alkyl group, and more preferably a methyl group (i.e. $C_1$ alkyl group).

In one preferred embodiment, the $R^1$ group represents an hydrogen atom.

<u>The $R^2$ group</u>

$R^2$ represents an hydrogen atom, a $C_1$-$C_5$ alkyl group, a group responding to formula (II) as defined in the present invention or a group responding to formula (III) as defined in the present invention.

The $C_1$-$C_5$ alkyl group as the $R^2$ group is preferably a linear $C_1$-$C_5$ alkyl group, and more preferably a methyl group.

The group responding to formula (II): $-(CH_2)_n-NH_2$ (II), where $3 \leq n \leq 18$ as the $R^2$ group is preferably such that $4 \leq n \leq 15$, and more preferably such that $5 \leq n \leq 10$.

The group responding to the formula (III) as defined in the present invention as the $R^2$ group is defined by the groups T, A, X and Q.

T represents a $C_1$-$C_{17}$ alkylene group, a $C_1$-$C_{17}$ alkenylene group, or a $C_1$-$C_{17}$ alkynylene group, and preferably a $C_1$-$C_{17}$ alkylene group.

An alkylene group is a bivalent saturated aliphatic hydrocarbon radical.

The $C_1$-$C_{17}$ alkylene group can be a branched or a linear alkylene group, and preferably a linear alkylene group. The alkylene group is preferably a $C_1$-$C_{10}$ alkylene group, and more preferably a $C_1$-$C_6$ alkylene group.

An alkenylene group is a bivalent insaturated aliphatic hydrocarbon radical comprising one or more double bonds C=C.

The $C_1$-$C_{17}$ alkenylene group can be a branched or linear (straight-chain) alkenylene group, and preferably a linear alkenylene group. The alkenylene group is preferably a $C_2$-$C_6$ alkenylene group, and more preferably a $C_2$-$C_4$

alkenylene group.

An alkynylene group is a bivalent insaturated aliphatic hydrocarbon radical comprising one or more triple bonds C≡C.

The $C_1$-$C_{17}$ alkynylene group can be a branched or linear (straight-chain) alkynylene group, and preferably a linear alkynylene group. The alkynylene group is preferably a $C_2$-$C_6$ alkynylene group, and more preferably a $C_2$-$C_4$ alkynylene group.

According to one particularly preferred embodiment, at least one of $R^1$ ou $R^2$ group is different from an hydrogen atom, and advantageously, the group $R^2$ is different from an hydrogen atom (in other word, the group $R^2$ represents advantageously an $C_1$-$C_5$ alkyl group, a group responding to formula (II) or a group responding to the following formula (III)).

The Q group

The alkyl group optionally substituted with a functional group as a Q group can be a linear or a branched alkyl group, and preferably a linear alkyl group.

The alkyl group optionally substituted with a functional group as a Q group can be a non-susbtituted $C_1$-$C_4$ alkyl group or an alkyl group comprising at least 5 carbon atoms and being optionally substituted with a functional group.

The alkyl group optionally substituted with a functional group as a Q group is preferably a $C_5$-$C_{20}$ alkyl group optionally substituted with a functional group, and more preferably a $C_6$-$C_{18}$ alkyl group optionally substituted with a functional group.

The functional group

The functional group can be selected from the following groups: amide, sulfonate, sulfate, quaternaty ammonium, hydroxyl, phosphonate, succinimidyl ester, sulfosuccinimidyl ester, isothiocyanate, isocyanate, iodoacetamide, maleimide, sulfonyl halide, acid halide (e.g. preferably acid chloride and acid bromide), carbodiimide, biotin, azido, alkyne, and a -C(O)-Z group, where Z is selected from an -OH, -N-glycine, -N-lysine, -Y-L-$NH_2$, -Y-L-COOH and -Y-L-SH group, where Y is selected from N and O atoms, and L is selected from a $C_1$-$C_{17}$ alkylene group, a $C_1$-$C_{17}$ alkenylene group, a $C_1$-$C_{17}$ alkynylene group, and a phenylene group.

The $C_1$-$C_{17}$ alkylene, $C_1$-$C_{17}$ alkenylene, and $C_1$-$C_{17}$ alkynylene groups are as defined in the present invention.

The phenylene group can be substituted with one or more W groups selected from hydroxyl, ether, primary and secondary amine, carboxylic acid, amide, nitro, and thiol group.

The functional group can either provide solubility, lipophilicity or improved relaxivity (e. g. amide, quaternary ammonium).

It can also be a recognition function which can interact with biological molecules of interest (e.g. biotin which interact with streptavidin or aromatic compounds with human serum albumin (HSA)) or a reactive function which is capable of reacting with a biological molecule to form a target molecule thatprovides a specific affinity fora given compound in a medium. Applications of such target molecules in the field of MRI are blood-pool agents (BPAs) for magnetic resonance angiography which remain confined to the intravascular space due to their high affinity with the seric protein HAS or molecular resonance (MR) contrast agents which target a specific protein, cell type or biological process. Application of such target molecules in the field of nuclear medicine are peptide-conjugates or immunoradiotracors for immunotherapy and immuno-PET imaging. Such agents are specificatly directed to the cell/protein of interest and have revolutionized treatment strategies for several types of cancer.

The biological molecule can be a sugar, a peptide, a protein, a nucleotide or an antibody.

The $NHR^{14}$ group as the Q group is preferably such that $R^{14}$ represents an aryl group optionally substituted with a functional group, said functional group being as defined in the present invention.

The aryl group as $R^{14}$ can be a $C_5$-$C_{20}$ aryl group optionally substituted with a functional group, and preferably a $C_6$-$C_{12}$ aryl group optionally substituted with a functional group.

The aryl group can be a phenyl group, a pyridine, a pyrimidine, a triazine, a thiophene, a porphyrine, an aryl-based cyanine or another aryl-based dye, and preferably a phenyl group.

The phenyl group can be substituted with one or more W groups (W being different from the functional group) selected from hydroxyl, ether, primary and secondary amine, carboxylic acid, amide, nitro, and thiol groups.

The polyethylene glycol group responding to formula (IV): $-CH_2-(CH_2-O-CH_2)_q-CH_2OH$ (IV), where $1 \leq q \leq 24$, as the Q group is preferably such that $1 \leq q \leq 12$.

In one preferred embodiment, $R^2$ represents a group responding to formula (II) or (III) as defined in the present invention, and more preferably a group responding to formula (III).

Indeed, the introduction of a group of formula (II) or (III) at the N7 position of the bispidine scaffold enables the formation of Mn(II) complexes with a sufficient thermodynamic stability and an extremely high kinetic inertness to be

used as MRI contrast agents and allows to modulate the physicochemical properties of the bispidine and its associated metal complexes, as well as their their biodistribution and biotoxicological properties.

The following embodiments as a group of formula (III) are particularly advantageous:

- A represents -NH-, X represents an oxygen atom, and Q represents an alkyl group optionally substituted with a functional group;
- A represents -NH-, X represents a sulfur atom, and Q represents a $NHR^{14}$ group where $R^{14}$ represents an aryl group optionally substituted with a functional group; or
- A represents $-CH_2-$, X represents an oxygen atom, and Q represents an OH group.

The $R^3$ and $R^4$ groups

$R^3$ and $R^4$, which may be identical or different, represent a $CH_2OH$ group, a $CO_2H$ group, or a $CONHR^{15}$ group, and preferably a $CH_2OH$ group or a $CO_2H$ group.

The alkyl group optionally substituted with a functional group as $R^{15}$ can be a linear or a branched alkyl group, and preferably a linear alkyl group.

The alkyl group optionally substituted with a functional group as $R^{15}$ is preferably a $C_5$-$C_{20}$ alkyl group optionally substituted with a functional group, and more preferably a $C_6$-$C_{18}$ alkyl group optionally substituted with a functional group.

The functional group is as defined in the present invention

The aryl group optionally substituted with a functional group as $R^{15}$ can be selected from a $C_5$-$C_{20}$ aryl group optionally substituted with a functional group, and preferably a $C_6$-$C_{12}$ aryl group optionally substituted with a functional group.

The aryl group as $R^{15}$ can be a phenyl group, a pyridine, a pyrimidine, a triazine, a thiophene, a porphyrine, an aryl-based cyanine, and another aryl-based dye, and preferably a phenyl group.

The phenyl group can be substituted with one or more W groups selected from hydroxyl, ether, primary and secondary amine, carboxylic acid, amide, nitro, and thiol groups.

The polyethylene glycol group responding to formula (V): $-CH_2-(CH_2-O-CH_2)_r-CH_2OH$ (V), where $1 \leq r \leq 24$ as $R^{15}$ is preferably such that $1 \leq r \leq 12$.

The $R^5$ group

$R^5$ represents an hydrogen atom, an alkyl group, a group of formula (VI): $-(CH_2)_m-CO_2H$ (VI) where $1 \leq m \leq 5$, a polyethylene glycol group responding to formula (VII): $-CH_2-(CH_2-O-CH_2)_s-CH_2OH$ (VII), where $1 \leq s \leq 18$, or a group of formula (III) as defined above.

The alkyl group as $R^5$ can be a linear or a branched alkyl group, and preferably a linear alkyl group.

The alkyl group as $R^5$ is preferably a $C_1$-$C_5$ alkyl group, and more preferably a methyl group.

The group of formula (VI): $-(CH_2)_m-CO_2H$ (VI) where $1 \leq m \leq 5$ as $R^5$ is preferably such that $1 \leq m \leq 3$.

The polyethylene glycol group responding to formula (VII): $-CH_2-(CH_2-O-CH_2)_s-CH_2OH$ (VII), where $1 \leq s \leq 18$ as $R^5$ is preferably such that $1 \leq s \leq 10$.

The group of formula (III) as $R^5$ is as defined in the present invention for $R^2$.

In one preferred embodiment, $R^5$ represents an alkyl group or a group of formula (VI): $-(CH_2)_m-CO_2H$ (VI) where $1 \leq m \leq 5$.

The $R^6$, $R^7$, $R^8$, $R^9$, $R^{10}$, $R^{11}$. $R^{12}$, and $R^{13}$ groups

The alkyl group as $R^{16}$ can be a linear or a branched alkyl group, and preferably a linear alkyl group.

The alkyl group as $R^{16}$ is preferably a $C_1$-$C_{10}$ alkyl group, and more preferably a $C_1$-$C_5$ alkyl group, and even more preferably a methyl or ethyl group.

The alkyl group optionally substituted with a functional group as $R^{17}$ can be a linear or a branched alkyl group, and preferably a linear alkyl group.

The alkyl group optionally substituted with a functional group as $R^{17}$ is preferably a $C_5$-$C_{20}$ alkyl group optionally substituted with a functional group, and more preferably a $C_6$-$C_{18}$ alkyl group optionally substituted with a functional group.

The functional group is as defined in the present invention

The aryl group optionally substituted with a functional group as $R^{17}$ can be selected from a $C_5$-$C_{20}$ aryl group optionally substituted with a functional group, and preferably a $C_6$-$C_{12}$ aryl group optionally substituted with a functional group.

The aryl group as $R^{17}$ can be a phenyl group, a pyridine, a pyrimidine, a triazine, a thiophene, a porphyrine, an aryl-based cyanine, and another aryl-based dye, and preferably a phenyl group.

The phenyl group can be substituted with one or more W groups (W being different from the functional group) selected from hydroxyl, ether, primary and secondary amine, carboxylic acid, amide, nitro, and thiol groups.

In one preferred embodiment, $R^6$, $R^7$, $R^8$, $R^9$, $R^{10}$, $R^{11}$, $R^{12}$, and $R^{13}$ groups represent hydrogen atoms or at least $R^7$, $R^9$, $R^{11}$, and $R^{13}$ represent hydrogen atoms. In other words, the pyridine rings can be substituted in 4 and/or 6 positions (i.e. at least one of $R^6$, $R^8$, $R^{10}$, and $R^{12}$ is different from an hydrogen atom).

In a preferred embodiment, which can be advantageously combined with the preceding preferred embodiment, $R^6$ and $R^{10}$ are identical, $R^7$ and $R^{11}$ are identical, $R^8$ and $R^{12}$ are identical, and $R^9$ and $R^{13}$ are identical.

In one particularly preferred embodiment, the bispidine-based metal chelating ligand of the invention is preferably selected from the following formulae (I-a) to (I-g):

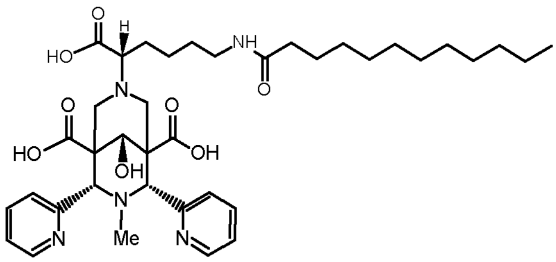

(I-a)

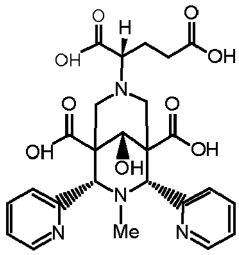

(I-b)

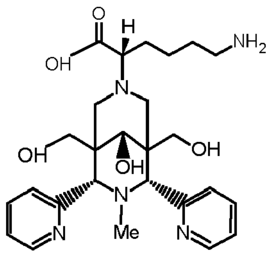

(I-c)

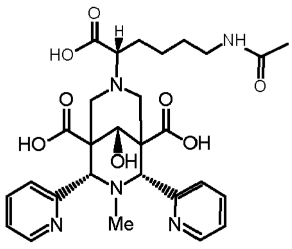

(I-d)

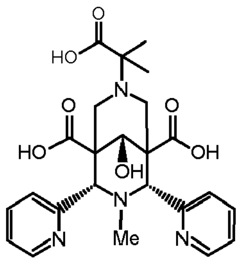

**(I-e)**

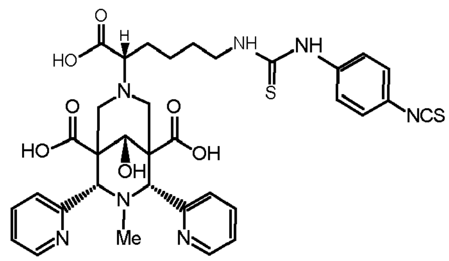

**(I-f)**

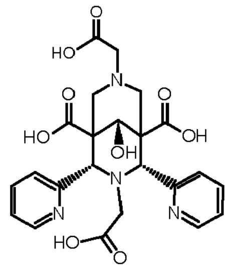

**(I-g)** .

A second object of the present invention is a complex of a metal M, wherein said complex comprises a metal ion of said metal M complexed with a bispidine-based metal chelating ligand (I) as defined in the first object of the present invention, said metal M being selected from copper, manganese, gallium, cobalt, zinc, nickel, and iron, and preferably selected from copper and manganese.

The metal M can be radioactive.

The metal ion can be selected from $Mn^{2+}$, $Mn^{3+}$, $Cu^{2+}$, $Cu^{+}$, $Ga^{3+}$, $Co^{2+}$, $Co^{3+}$, $Fe^{2+}$, $Fe^{3+}$, $Zn^{2+}$, or $Ni^{2+}$.

The complexes of the present invention exhibit high relaxivity, show a favorable pharmacokinetic profile, are completely excreted, are chemically stable, exhibit high water solubility, offer the possibility of providing safe alternatives to traditional GBCAs and offer the potential for a significant dose reduction in comparison to state-to-the-art Mn-based CAs, and are suitable for imaging of different body regions.

A third object of the present invention is the use of a bispidine-based metal chelating ligand (I) as defined in the first object of the present invention or a complex as defined in the second object of the present invention, in the field of medical imaging or therapy, and more specifically as MRI (magnetic resonance imaging) contrast agents and/or nuclear imaging agents for PET (positron emission tomography) or SPECT (single photon emission tomography).

Positron emission tomography (PET) is a highly sensitive imaging technique with high tissue penetration. This technique can allow for the *in vivo* imaging of diseased tissues by targeting biochemical processes; thus allowing for detection of disease before physical changes occur.

More particularly, the bispidine-based metal chelating ligand (I) as defined in the first object of the present invention can be used as a chelator for radioactive copper for application in immuno-positron emission tomography (PET).

More particularly, the bispidine-based metal chelating ligand (I) as defined in the first object of the present invention can be used as bifunctional chelating agents for HER2+ immuno-imaging of breast cancer or for PET immuno-imaging of multiple myeloma, for radioimmunotherapy or again as a theranostic probe combining therapy and diagnostic.

The present invention is illustrated in more detail in the examples below, but it is not limited to said examples.

## Examples

Solvents and starting materials were purchased from Aldrich, Acros and Alfa Aesar and used without further purification. IR spectra were recorded on a Perkin Elmer Spectrum One Spectrophotometer as solid samples and only the most significant absorption bands are given in $cm^{-1}$. Elemental analyses and mass spectrometry analysis were carried out by the Service Commun d'Analyses of the University of Strasbourg. $^{1}H$ and $^{13}C$ NMR spectra and 2D COSY, NOESY, HSQC, and HMBC experiments were recorded on Avance 300 and Avance 400 spectrometers operating at 7.04 T and 9.39 T, respectively. Chemical shifts are reported in ppm, with residual protonated solvent as internal reference.

Example 1: synthesis of intermediate compound 4

Compound 4 was prepared according to the following scheme 2:

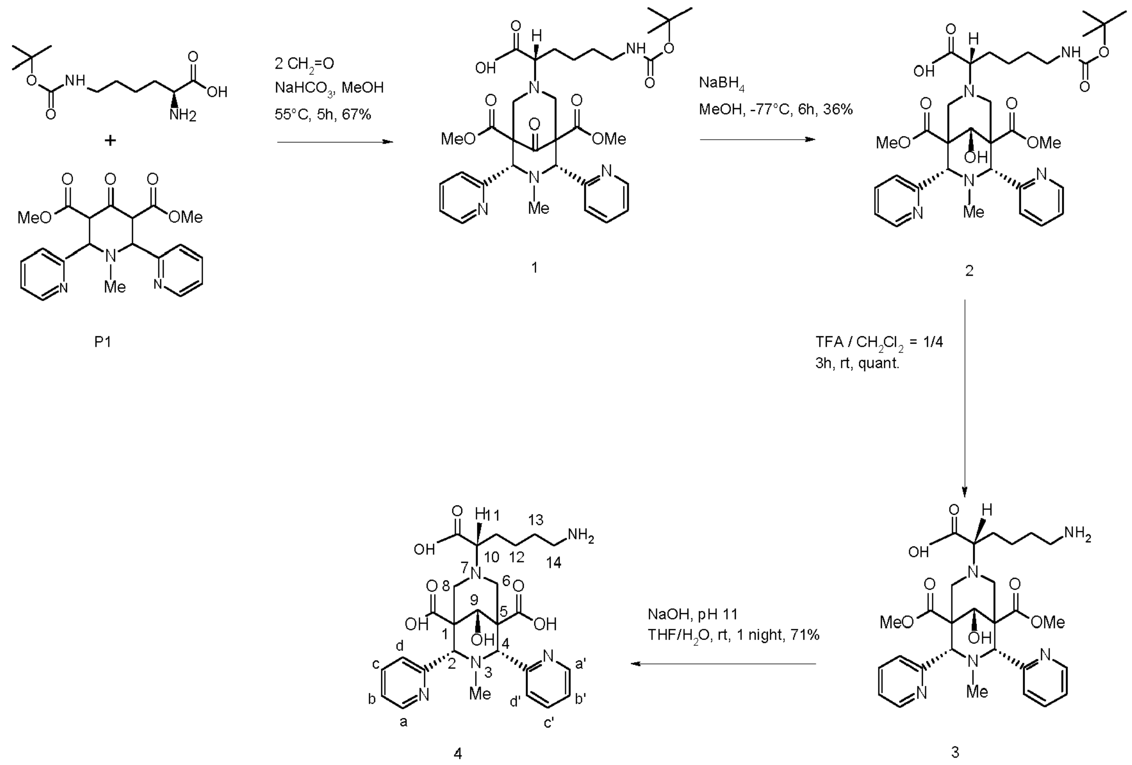


**SCHEME 2**

**P1** is a well-known starting material to lead to bispidone and bispidine compounds.

*Bispidone **1**.* In a solution of methanol (60 mL), Nε-Boc-L-lysine (4.19 g, 17.40 mmol) and $NaHCO_3$ (1.46 g, 17.40 mmol) were mixed under stirring during 1h at 45°C. Then, formaldehyde (3.5 mL, 47.50 mmol) and precursor **P1** (6.06 g, 15.80 mmol) in methanol (20 mL) were added at room temperature (rt) and the mixture was stirred under reflux during 5h. At the end of the reaction, the solution was filtered and solvents were removed under reduced pressure. The obtained solid was dissolved in a minimum of methanol and was precipitated upon dropwise addition in a large amount of diethyl ether. After centrifugation, precipitates were collected and dried to give a pure beige solid (6.93 g, 67%).

TLC ($Al_2O_3$; DCM/MeOH, 85/15); R*f* = 0.5.

$^{1}H$-NMR (400 MHz, $CDCl_3$): δ 8.95 (m, 2H, Ha+Ha'), 7.70 (t, *J*= 7.6 Hz, 1H, Hc'), 7.59 (t, *J*= 7.5 Hz, 1H, Hc), 7.35 (m, 1H, Hb'), 7.17 (m, 3H, Hd'+Hd+Hb), 4.67 (s, 1H, H2), 4.56 (s, 1H, H4), 3.81 (broad s, 1H, COOH), 3.77 (s, 3H, $OCH_3$), 3.66 (s, 3H, $OCH_3$), 3.22 (AB system, $\delta_A$ = 3.60, $\delta_B$ = 2.84, $J_{AB}$ = 12.0 Hz, $v_A$ = 1440 Hz, $v_B$ = 1136 Hz, 2H, H8/H6), 2.96 (AB system, $\delta_A$ = 3.29, $\delta_B$ = 2.62, $J_{AB}$ = 12.0 Hz, $v_A$ = 1316 Hz, $v_B$ = 1048 Hz, 2H, H8/H6), 2.99 (m, 2H, H14), 2.57 (d, 1H, H10), 1.86 (s, 3H, $CH_3$), 1.64-1.22 (m, 15H, H11/H12/H13/$OC(CH_3)_3$).

$^{13}C$-NMR (100 MHz, $CDCl_3$): δ 202.7 (C9), 177.0 (COOH), 168.0 (COOMe), 167.5 (COOMe), 156.1 (3C, 1Cpy+Cpy'+COO*t*Bu), 151.5 (Ca/Ca'), 151.1 (Ca/Ca'), 137.5 (Cc/Cc'), 137.0 (Cc/Cc'), 124.5 (Cb/Cb'/Cd/Cd'), 124.2 (Cb/Cb'/Cd/Cd'), 124.0 (Cb/Cb'/Cd/Cd'), 121.7 (Cb/Cb'/Cd/Cd'), 78.7 ($C(OCH_3)_3$), 74.0 (C10), 72.6 (2C, C2+C4), 63.0

(2C, C1+C5), 57.5 (C6/C8), 54.7 (C6/C8), 52.8 ($OCH_3$), 52.5 ($OCH_3$), 43.0 ($CH_3$), 40.3 (C14), 29.9 (C11), 28.9 (C13), 28.5 ($C(CH_3)_3$), 24.0 (C12).

IR ($cm^{-1}$, ATR) ν 3366 (broad, N-H amide), 2943, 2860 (broad, O-H acid), 1739 (s, C=O ester), 1695 (s, C=O acid), 1572 (s, C=C aromatic), 1254 (s, CO).

Electrospray ionization ESI/$MS^+$: *m/z* = 654.31 ($[M+H]^+$, 100%), 655.31 ($[M+H]^+$, 35.7%), 656.32 ($[M+H]^+$, 6.2%), 657.32 ($[M+H]^+$, 0.7%).

*Bispidol* ***2.*** Bispidone **1** (3.08 g, 4.71 mmol) was dissolved in anhydrous methanol (110 mL) and was cooled at -77°C on a bath of acetone/dry ice. Then, sodium borohydride (240.00 mg, 7.07 mmol) was slowly added. After 6h at -77°C, the mixture was placed at 4°C for one night. Then $NH_4Cl$ (520.00 mg, 9.72 mmol) was added in the flask and the mixture was stirred during 10 minutes. Solvents were removed under reduced pressure and the obtained solid was purified by flash chromatography column (reverse phase, $CH_3CN/H_2O$ 20/80-25/75). The pure compound **2**.$2H_2O$ was obtained as a white solid (1.10 g, 32%).

TLC ($SiO_2$; DCM/MeOH, 80/20); R*f* = 0.3-0.5.

$^1H$-NMR (400 MHz, MeOD): δ= 8.67 (m, 2H, Ha+Ha'), 7.81 (m, 2H, Hc+Hc'), 7.60 (m, 2H, Hd+Hd'), 7.38 (m, 2H, Hb+Hb'), 4.89 (s, 2H, H2+H4), 4.50 (s, 1H, H9), 4.30 (AB system, $\delta_A$ = 4.60, $\delta_B$ = 4.00, $J_{AB}$ = 12.0 Hz, 2H, H6/H8), 3.96 (AB system, $\delta_A$ = 4.10, $\delta_B$ = 3.91, $J_{AB}$ = 12.0 Hz, 2H, H6/H8), 3.76 (m, 1H, H10), 3.63 (s, 3H, $OCH_3$), 3.55 (s, 3H, $OCH_3$), 3.08 (m, 2H, H14), 2.33-2.17 (m, 2H, H11), 1.73 (m, 4H, H12+H13), 1.65 (s, 3H, $CH_3$), 1.39 (s, 9H, $C(CH_3)_3$).

$^{13}C$-NMR (75 MHz, MeOD): δ 172.8 (COOH), 170.4 (2C, COOMe), 158.3 (COO*t*Bu/Cpy/Cpy'), 157.8 (COO*t*-Bu/Cpy/Cpy'), 157.7 (COO*t*Bu/Cpy/Cpy'), 150.6 (Ca/Ca'), 150.1 (Ca/Ca'), 138.1 (Cc/Cc'), 138.0 (Cc/Cc'), 128.6 (2C, Cd+Cd'), 124.9 (Cb/Cb'), 124.8 (Cb/Cb'), 79.7 ($C(CH_3)_3$), 73.8 (C9), 72.0 (C10), 67.8 (C2/C4), 67.5 (C2/C4), 56.8 (C8/C6), 53.0 ($OCH_3$), 52.9 ($OCH_3$), 52.3 (2C, C1+C5), 51.2 (8/6), 41.6 ($CH_3$), 41.1 (C14), 30.7 (C13), 30.0 (C11), 28.7 ($C(CH_3)_3$), 25.1 (C12).

Electrospray ionization ESI/$MS^+$: *m/z* = 656.33 ($[M+H]^+$, 100%), 657.34 ($[M+H]^+$, 35.7%), 658.34 ($[M+H]^+$, 3.5%).

Elemental analysis calculated for $C_{33}H_{45}O_9N_5$.$1.5H_2O$: C, 58.05, H, 7.09, N, 10.26. Found: C, 57.82, H, 6.81, N, 10.38.

*Bispidol* ***3.*** Bispidol **2** (413.0 mg, 0.74 mmol) was dissolved in $CH_2Cl_2$ (8 mL) and trifluoroacetic acid (2 mL) and the mixture was stirred at room temperature. After 3h, solvents were evaporated under reduced pressure and the crude TFA salt was used in the next step without purification (413 mg).

TLC ($C_{18}$; $CH_3CN/H_2O$, 0.1% TFA, 50/50); R*f* = 0.8.

$^1H$-NMR (400 MHz, MeOD): δ 8.76 (m, 2H, Ha+Ha'), 7.94 (m, 2H, Hc+Hc'), 7.77 (m, 2H, Hd+Hd'), 7.52 (m, 2H, Hb+Hb'), 5.63 (s, 1H, H2/H4), 5.61 (s, 1H, H2/H4), 4.41 (s, 1H, H9), 3.72 (s, 3H, $OCH_3$), 3.69 (s, 3H, $OCH_3$), 3.33 (AB system, $\delta_A$ = 3.52, $\delta_B$ = 3.13, $J_{AB}$ = 12.0 Hz, $v_A$ = 1408 Hz, $v_B$ = 1252 Hz, 2H, H6/H8), 3.39 (AB system, $\delta_A$ = 3.41, $\delta_B$ = 3.38, $J_{AB}$ = 10.5 Hz, $v_A$ = 1363 Hz, $v_B$ = 1353 Hz, 1H, H10), 3.13 (m, 2H, H6/H8), 3.03 (t, *J*= 8.5Hz, 2H, H14), 2.35-1.92 (m, 2H, H11), 2.31 (s, 3H, $CH_3$), 1.90 (m, 2H, H13), 1.61 (m, 2H, H12).

$^{13}$CNMR (75 MHz, $D_2O$): δ 173.6 (COOH), 170.5 (COOMe), 170.4 (COOMe), 152.9 (Cpy/Cpy'), 152.8 (Cpy/Cpy'), 151.0 (Ca/Ca'), 150.4 (Ca/Ca'), 139.1 (Cc/Cc'), 139.0 (Cc/Cc'), 129.0 (Cd/Cd'), 128.6 (Cd/Cd'), 126.2 (Cb/Cb'), 126.1 (Cb/Cb'), 73.9 (C9), 68.1 (C10), 67.6 (C2/C4), 67.1 (C2/C4), 55.8 (C6/C8), 54.4 (C1/C5), 53.7 (C1/C5), 53.3 ($OCH_3$), 53.2 ($OCH_3$), 50.4 (C6/C8), 43.4 ($CH_3$), 40.7 (C14), 29.8 (C11), 28.8 (C13), 25.0 (C12).

*Compound* ***4.*** Bispidol **3** (279.0 mg, 0.50 mmol) was dissolved in a mixture of $H_2O$/THF (8 mL/2 mL) and sodium hydroxide (240.0 mg, 3.0 mmol) was added at room temperature under stirring. After 48h, the mixture was evaporated under reduced pressure and was purified by column chromatography (reverse phase, 100% $H_2O$ to 100% MeOH in 15 min).

Pure compound 4.2Na (265.0 mg, 71%) was obtained as a white powder.

TLC ($C_{18}$; $CH_3CN/H_2O$, 20/80), R*f* = 0.4.

$^1H$-NMR (400 MHz, $D_2O$): δ 8.48 (m, 2H, Ha+Ha'), 7.54 (m, 2H, Hc+Hc'), 7.30 (d, *J*= 9.0 Hz, 1H, Hd/Hd'), 7.19 (m, 1H, Hd/Hd'), 7.14 (m, 2H, Hb+Hb'), 4.40 (s, 1H, H2/H4), 4.31 (s, 1H, H2/H4), 3.82 (s, 1H, H9), 2.42 (AB system, $\delta_A$ = 2.92, $\delta_B$ = 1.93, $J_{AB}$ = 12.3 Hz, $v_A$ = 1167 Hz, $v_B$ = 774 Hz, 2H, H6/H8), 2.36 (t, *J*= 6.9 Hz, 2H, H14), 2.29 (AB system, $\delta_A$ = 2.75, $\delta_B$ = 1.83, $J_{AB}$ = 11.2 Hz, $v_A$ = 1102 Hz, $v_B$ = 731 Hz, 2H, H6/H8), 2.20 (m, 1H, H10), 1.51 (s, 3H, CH3), 1.47-1.36 (m, 2H, H11), 1.20 (m, 2H, H13), 1.10 (m, 2H, H12).

$^{13}C$-NMR (100 MHz, $D_2O$): δ 181.4 (COOH), 179.1 (COOH), 178.6 (COOH), 159.9 (Cpy/Cpy'), 159.3 (Cpy/Cpy'), 149.2 (2C, Ca+Ca'), 137.0 (2C, Cc+Cc'), 126.0 (Cd/Cd'), 125.5 (Cd/Cd'), 122.9 (Cb/Cb'), 122.8 (Cb/Cb'), 75.3 (C10), 74.3 (C9), 67.8 (C2/C4), 67.1 (C2/C4), 55.9 (C6/C8), 53.3 (C5/C8), 51.7 (C1/C5), 51.5 (C1/C5), 42.8 (CH3), 40.2 (C14), 32.0 (C13), 29.6 (C11), 23.0 (C12).

IR ($cm^{-1}$, ATR) ν 3342 (broad, O-H alcool/N-H amine), 2949 (broad, O-H acid), 1584, 1568 (s, C=C aromatic/N-H amine), 1366 (s, C-O).

Electrospray ionization ESI/$MS^+$: *m/z* = 528.25 ($[M+H]^+$, 100%), 529.25 ($[M+H]^+$, 28.1%), 530.25 ($[M+H]^+$, 3.8%).

Elemental analysis calculated for $C_{26}H_{31}O_7N_5Na2.3H_2O$: C, 49.91, H, 5.96, N, 11.19. Found: C, 49.79, H, 5.92, N, 11.19.

Example 2: synthesis of compound (I-a)

Compound (I-a) was prepared according to the following scheme 3:

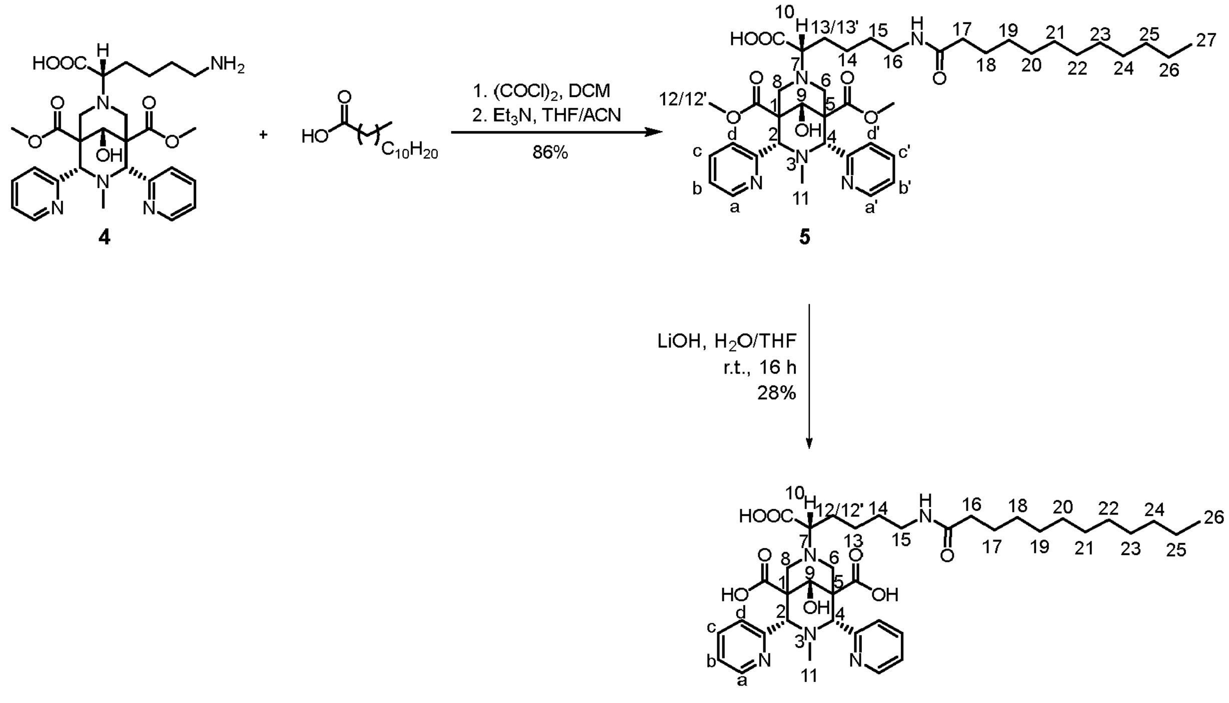


**4** **5** **(I-a)**

## SCHEME 3

*Compound* ***5.*** Oxalyl chloride (58 µl, 0.68 mmol, 3 eq) is dissolved in a solution of dodecanoic acid (67 mg, 0.33 mmol, 1.5 eq) at 0 °C under argon. The mixture is stirred at room temperature for 3 hours. Then, the solvent is distilled at the vacuum ramp fitted with a trap and the colourless oil is dissolved in a mixture of ACN (4 ml) and THF (4 ml) to form a mixture. Compound **4** (150 mg, 0.22 mmol, 1 eq) and $Et_3N$ (94 µl, 0.67 mmol, 3 eq) are added to said mixture and the resulting solution is stirred at room temperature for 16 hours. The solvent is then removed under vacuum and the crude product is purified by FPLC ($C_{18}$, 97/3 $H_2O$/ACN to 50/50 $H_2O$/ACN) to give **5** (140 mg) with a 86 % yield.

$^1H$ NMR (MeOD, 400 MHz) : δ 0.90 (t, *J* = 6.9 Hz, 3H, H27); 1.29 (m, 16H, H19-26); 1.63 (m, 4H, H14/H15 + H18); 1.74 (m, 2H, H14/H15); 1.89 (m, 1H, H13/13'); 2.21 (t, *J* = 7.4 Hz, 2H, H17); 2.31 (m, 4H, H13/13' + H11); 3.13 (m, 3H, H6/H8); 3.29 (m, 2H, H16); 3.39 (dd, $J_1$ = 10.0 Hz, $J_2$ = 4.5 Hz, 1H, H10); 3.57 (m, 1H, H6/8); 3.70 (s, 3H, H12/12'); 3.74 (s, 3H, H12/12'); 4.41 (s, 1H, H9); 5.63 (s, 1H, H2/4); 5.64 (s, 1H, H2/4); 7.53 (m, 2H, Hb/b'); 7.77 (m, 2H, Hd/d'); 7.95 (td, $J_1$ = 7.8 Hz, $J_2$ = 1.8 Hz, 2H, Hc/c'); 8.79 (m, 2H, Ha/a').

$^{13}C$ NMR (MeOD, 126 MHz): δ 13.08; 22.34; 24.03; 25.77; 28.56; 28.95; 29.07(2 $CH_2$); 29.22; 29.27; 29.34 (2 $CH_2$); 31.76; 35.84; 38.88; 41.92; 48.87; 51.84; 51.88; 51.95; 52.32; 54.63; 65.74; 66.28; 66.93; 72.58; 124.71; 124.76; 127.28; 127.61; 137.51; 137.63; 149.11; 149.64; 151.51; 151.63; 166.94; 169.08; 172.32; 174.96.

Mass Spectrometry: ($ESI^+$/MS): m/z = 738.43 $[M + H]^+$, calculated for $C_{40}H_{59}N_5O_8$: 737.44.

*Compound* ***(I-a).*** Lithium hydroxide (23 mg, 0.95 mmol, 5.3 eq) is dissolved in a solution of compound **5** (140 mg, 0.18 mmol, 1 eq) in THF (6 ml) and $H_2O$ (14 ml). The mixture is stirred at room temperature for 2 days. At the end, the solvent is removed under vacuum, the crude product is dissolved in a minimum of water and the pH is lowered to 2. The solution is purified by FPLC ($C_{18}$, 97/3 $H_2O$/ACN to 100 % ACN) to give compound **(I-a)**.2.5LiCl (48 mg) with a 28 % yield.

$^1H$ NMR (MeOD, 400 MHz) : δ 0.9 (t, *J* = 7.2 Hz, 3H, H26); 1.29 (m, 16H, H18-25); 1.63 (m, 4H, H13/H14 + H17); 1.74 (m, 2H, H13/H14); 1.89 (m, 1H, H12/12'); 2.21 (m, 2H, H16); 2.31 (m, 4H, H12/12' + H11); 3.11 (m, 3H, H6/H8); 3.30 (m, 2H, H15); 3.36 (m, 1H, H10); 3.57 (m, 1H, H6/8); 4.43 (s, 1H, H9); 5.58 (s, 1H, H2/4); 5.59 (s, 1H, H2/4); 7.53 (m, 2H, Hb); 7.77 (m, 2H, Hd); 7.95 (td, $J_1$ = 7.8 Hz, $J_2$ = 1.8 Hz, 2H, Hc); 8.79 (m, 2H, Ha).

Mass Spectrometry: ($ESI^+$/HRMS): m/z = 710.4113 $[M + H]^+$, calculated for $C_{38}H_{55}N_5O_8$: 709.41.

Elemental Analysis: Calculated for $C_{38}H_{55}N_5O_8$.2.5(LiCl), C, 55.94, H, 6.79, N, 8.58. Found: C,56.38, H, 6.71, N, 8.17.

Example 3: synthesis of compound (I-b)

Compound (I-b) was prepared according to the following scheme 4:

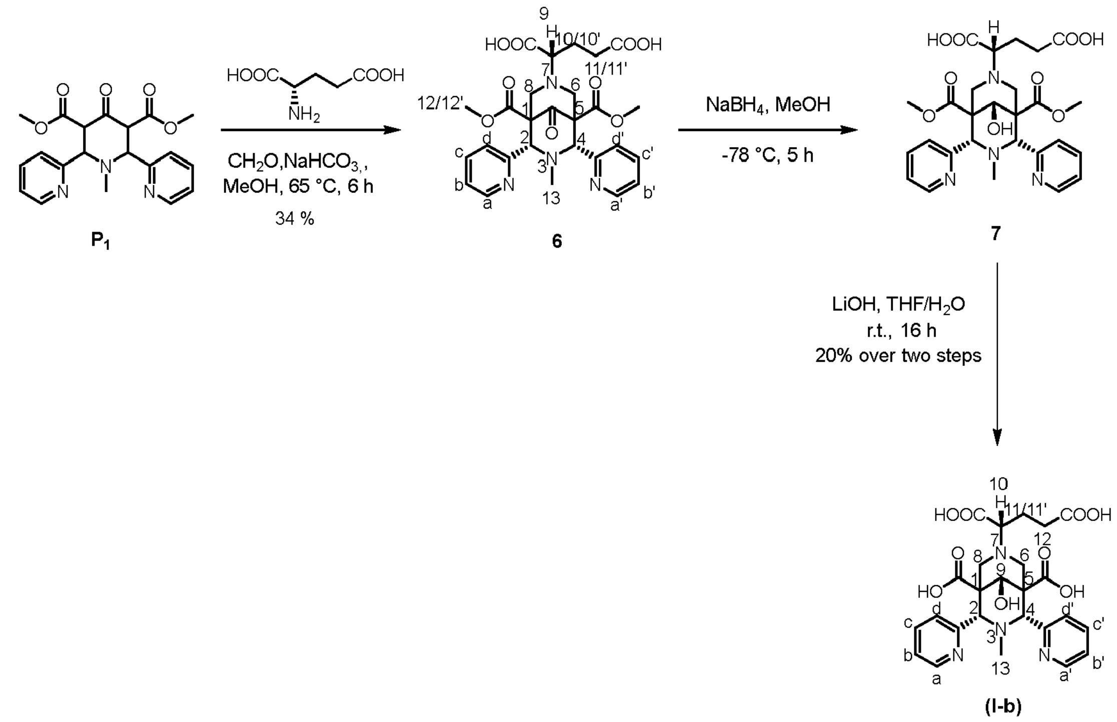


**SCHEME 4**

*Bispidone **6**.* L-glutamic acid (422 mg, 2.87 mmol, 1.1 eq), $NaHCO_3$ (241.5 mg, 2.87 mmol, 1.1 eq) and formaldehyde 37 % (635 μl, 8.52 mmol, 3.3 eq) are added to a solution of $\mathbf{P_1}$ (1 g, 2.61 mmol, 1 eq) in MeOH and the mixture is stirred at reflux for 2 days. At the end of the reaction, the solvent is removed under vacuum and the crude product is precipited in $CH_3CN$ to give bispidone **6** (500 mg) as a white powder with a 34 % yield.

$^1H$ NMR (MeOD, 400 MHz): δ 1.86 (m, 4H, H13 + H10/10'); 2.22 ppm (m, 1H, H10/10'); 2.24 (m, 1H, H11/11'); 2.37 (m, 1H, H11/11'); 2.56 (m, 2H, H6/8 + H9); 2.77 (m, 1H, H6/8); 3.40 (m, 1H, H6/8); 3.58 (m, 1H, H6/8); 3.69 (s, 3H, H12/12'); 3.76 (s, 3H, H12/12'); 4.65 (s, 1H, H2/4); 4.74 (s, 1H, H2/4); 7.34 (m, 2H, Hb/b'); 7.41 (m, 1H, Hd/d'); 7.44 (m, 1H, Hd/d'); 7.84 (m, 2H, Hc/c'); 8.84 (m, 2H, Ha/a').

$^{13}C$ NMR (MeOD, 126 MHz): δ 27.02 (C10); 33.87 (C11); 41.84 (C13); 51.66 (C12/12'); 51.86 (C12/12'); 55.62; (C6/8); 57.37 (C6/8); 62.75 ($C^4$); 62.82 ($C^4$); 72.24 (C2/4); 72.50 (C2/4); 73.92 (C9); 123.78 (Cb/b'); 124.10 (Cb/b'); 124.74 (Cd/d'); 124.86 (Cd/d'); 137.60 (Cc/c'); 137.83 (Cc/c') ; 150.40 (Ca/a'); 150.58 (Ca/a'); 155.96 ($C^4$); 156.21 ($C^4$); 167.34 ($C^4$); 167.70 ($C^4$); 177.59 ($C^4$); 179.36 ($C^4$); 202.15 ($C^4$).

*Compound **(I-b)**.* In a solution of bispidone **6** (500 mg, 0.90 mmol, 1 eq) in MeOH at -78 °C, sodium borohydride (32 mg, 0.85 mmol, 0.94 eq in total) is added three times (0.25 eq, 0.25 eq and then 0.5 eq the third time) at 1 hour of interval and the mixture is stirred at -78 °C for 2 hours. After two hours, the reaction is quenched with $NH_4Cl$ (5 ml) and the solvent is removed under vacuum. Then, DCM is added and the salts are filtered. The solvent is evaporated and the crude product is purified by reverse phase FPLC ($C_{18}$, $CH_3CN/H_2O$). The desired product can be identified in the NMR spectra but others species that was attribute to a partial hydrolysis of the methylester groups are present so the mixture is engaged in the deprotection step without any further purification. The isolated product (200 mg) is thus dissolved in a mixture of THF (3 ml) and $H_2O$ (7 ml). Lithium hydroxide (32 mg, 1.34 mmol) is added and the resulting mixture is stirred at room temperature for 16 hours. The reaction is monitored by TLC on $C_{18}$ using $MeOH/H_2O$ as eluent. At the end of the reaction, the solvent is removed under vacuum and the crude product is dissolved in a minimun of water to adjust the pH at 2. The solution is finally purified by FPLC ($C_{18}$, 97/3 $H_2O$/MeOH to 50/50 $H_2O$/MeOH) to give compound **(I-b)**.$2H_2O$ (100 mg) with a 20 % yield.

$^1H$ NMR (MeOD, 400 MHz): δ 2.11 (m, 1H, H11/11'); 2.21 (s, 3H, H13); 2.41 (m, 1H, H11/11'); 2.79 (m, 2H, H12); 2.95 (m, 1H, H6/8); 3.10 (m, 2H, H6/8); 3.36 (m, 1H, H6/8); 3.48 (m, 1H, H10); 4.3 (s, 1H, H9); 5.29 (s, 2H, H2/4); 7.57 (m, 2H, Hb/b'); 7.70 (m, 2H, Hd/d'); 7.99 (m, 2H, Hc/c'); 8.75 (m, 2H, Ha/a').

$^{13}C$ NMR ($D_2O$, 150 MHz): δ 24.53; 31.08; 41.73; 50.34; 51.05; 56.08; 63.03; 67.44; 67.68; 68.06; 73.65; 123.60 (2CH); 126.03; 126.28; 137.06 (2CH); 148.74; 149.11; 154.39; 154.58; 171.96; 173.39; 173.89; 175.83.

Mass Spectrometry: ($ESI^+$/MS): m/z = 529.19 $[M + H]^+$, calculated for $C_{25}H_{28}N_4O_9$: 528.19.

Elemental Analysis: Calculated for $C_{25}H_{28}N_4O_9.2(H_2O)$, C, 53.19, H, 5.71, N, 9.92. Found: C, 53.07, H, 5.59, N, 10.06.

Example 4: synthesis of compound (I-c)

Compound (I-c) was prepared according to the following scheme 5:

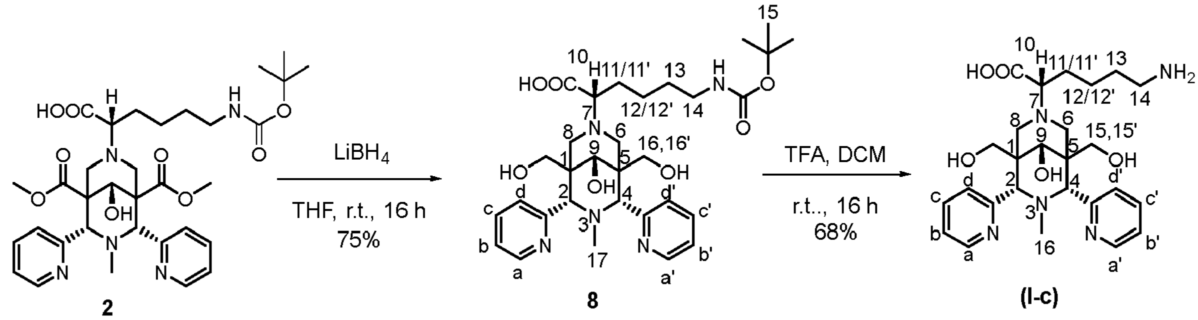


2 8 **(I-c)**

**SCHEME 5**

*Bispidol **8**.* In a solution of bispidol **2** (250 mg, 0.38 mmol, 1 eq) in 20 ml of THF and 2 ml of MeOH, 3 eq of $LiBH_4$ (82 mg, 3.76 mmol, 10 eq) are added in two times at 1 hour of interval and the mixture is stirred during 3 hours at room temperature. The reaction is monitored by TLC on $C_{18}$ using $H_2O$/MeOH (90/10) as eluent. At the end, the reaction is quenched with $NH_4Cl_{(aq)}$ (5 ml) and the solvents are removed under vacuum. DCM is added and the salts are filtered. The solvent is evaporated, and the crude product is purified by FPLC ($C_{18}$, 95/5 $H_2O$/ACN, to 100 % ACN) to give **8** (170 mg) with a 75 % yield.

$^1H$ NMR (MeOD, 400 MHz): δ 1.42 (m, 15H, H15 + H11/11' + H12/12' + H13); 1.72 (s, 3H, H17); 2.13 (AB system, $\delta_A$ = 1.67, $\delta_B$ = 2.6, $J_{AB}$ = 11.3 Hz, 2H, H6/$8_{ax}$ + H6/$8_{eq}$); 2.29 (dd, $J_1$ = 10.2 Hz, $J_2$ = 4.8 Hz, 1H, H10); 2,31 (AB system, $\delta_A$ = 1.82, $\delta_B$ =2.79, $J_{AB}$ = 11.3 Hz, 2H, H6/8ax + H6/8eq); 3.00 (m, 2H, H14); 3.16 (AB system, $\delta_A$ = 2.86 , $\delta_B$ = 3.45, $J_{AB}$ = 11.0 Hz, 2H, H16/16'); 3.32 (AB system, $\delta_A$ = 3.09 , $\delta_B$ = 3.56, $J_{AB}$ = 11.0 Hz, 2H, H16/16'); 4.07 (s, 1H, H2/4); 4.12 (s, 1H, H9); 4.13 (s, 1H, H2/4); 7.36 (m, 4H, Hb/b' + Hd/d'); 7.74 (m, 2H, Hc/c'); 8.71 (m, 2H, Ha/a').

$^{13}C$ NMR (MeOD, 126 MHz): δ23.37 ($CH_2$lys); 27.48(C15); 29.7($CH_2$-lys); 29.95 ($CH_2$-lys); 39.84 (C14); 41.93 ($C^4$); 42.56(C17 + $C^4$); 52.03 (C6/8); 54.99 (C6/8); 65.02 (C16/16'); 65.50 (C16/16'); 67.42 (C2/4); 68.15 (C2/4); 72.68 (C9); 75.23 (C10); 78.42 ($C^4$); 122.59 (Cb/b'); 122.95 (Cb/b'); 125.24 (Cd/d'); 125.70 ((Cd/d'); 136.44 (Cc/c'); 136.60 (Cc/c'); 149.81 (Ca/a'); 150.03 (Ca/a'); 157.10 ($C^4$); 158.98 ($C^4$); 159.63 ($C^4$); 179.55 ($C^4$).

Mass Spectrometry: ($ESI^+$/MS): m/z = 600.34 $[M + H]^+$, calculated for $C_{31}H_{45}N_5O_7$: 599.33.

*Compound **(I-c)**.* TFA (2 ml) is added to a solution of bispidol **8** (170 mg, 0.28 mmol, 1 eq) in 7 ml of DCM. The mixture is stirred for 16 hours at room temperature. At the end of the reaction, the solvent is removed under vacuum and the crude product is purified by FPLC ($C_{18}$, , 97/3 $H_2O$/ACN, 0.1 % TFA to 100 % ACN, 0.1 % TFA) to give compound **(I-c)**.3TFA (160 mg) with a 68 % yield.

$^1H$ NMR (MeOD, 400 MHz): δ 1.60 (m, 2H H11/11'); 1.90 (m, 3H, H12/12' + H13); 2.29 (m, 4H, H16 + H12/12'); 2.68 (AB system, $\delta_A$ = 2.59, $\delta_B$ = 2.77, $J_{AB}$ = 11.7 Hz, 2H, H6/8ax + H6/8eq); 2.80 (AB system, $\delta_A$ = 2.53 , $\delta_B$ = 3.07, $J_{AB}$ = 11.7 Hz, 2H, H6/8ax + H6/8eq); 3.03 (m, 2H, H14); 3.34 (AB system, $\delta_A$ = 3.12, $\delta_B$ = 3.55, $J_{AB}$ = 11.0 Hz, 2H, H15/15' + H15/15'); 3.37 (AB system, $\delta_A$ = 3.19, $\delta_B$ = 3.55, $J_{AB}$ = 11.0 Hz, 2H, H15/15' + H15/15'); 3.37 (dd, $J_1$ = 9.5 Hz, $J_2$ = 4.5 Hz, 1H, H10); 4.29 (s, 1H, H9); 5.12 (s, 1H, H2/4); 5.18 (s, 1H, H2/4); 7.56 (m, 4H, Hd/d' + Hb/b'); 7.94 (m, 2H, Hc/c'); 8.80 (m, 2H, Ha/a').

$^{13}C$ NMR (MeOD, 126 MHz) : δ 23.73 ($CH_2$lys); 27.42 ($CH_2$lys); 28.47 ($CH_2$lys); 39.32 (C14); 41.29 (C17); 42.74 ($C^4$); 43.11 ($C^4$); 48.60 (C6/8); 54.39 (C6/8); 63.57 (C15/15'); 63.70 (C15/15'); 66.69 (C10); 67.71 (C2/4); 68.18 (C2/4); 71.70 (C9); 124.47 (Cb/b'); 124.53 (Cb/b'); 125.52 (Cd/d'); 125.65 (Cd/d'); 137.57 (Cc/c'); 149.47 (Ca/a'); 150.00 (Ca/a'); 151.60 ($C^4$); 172.36 ($C^4$).

Mass Spectrometry: ($ESI^+$/MS): m/z = 500.29 $[M + H]^+$, calculated for $C_{26}H_{37}N_5O_5$: 499.28.

Elemental Analysis: Calculated for $C_{26}H_{37}N_5O_5$.3TFA: C, 45.80, H, 4.94, N, 8.48. Found: C, 45.66, H, 4.79, N, 8.32.

Example 5: synthesis of compound (I-d)

Compound (I-d) was prepared according to the following scheme 6:

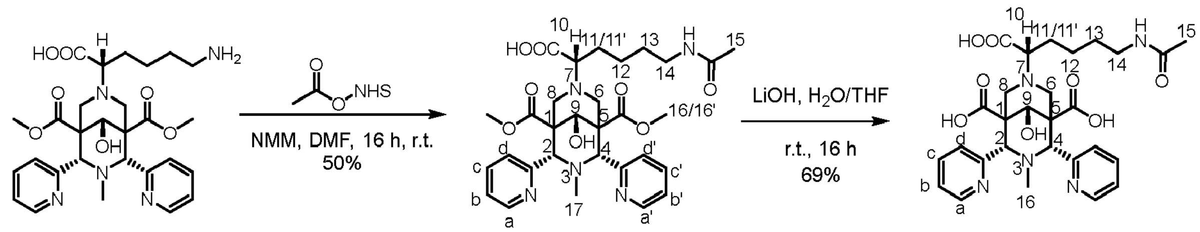


3 9 (I-d)

**SCHEME 6**

*Bispidol **9**.* N-methylmorpholine (132 μl, 1.2 mmol, 4 eq) and N-succinimidyl acetate (59.7 mg, 0.38 mmol, 1.3 eq) are added to a solution of bispidol **3** (200 mg, 0.3 mmol, 1 eq) in 10 ml of DMF. The mixture is stirred for 16 hours at room temperature. At the end, the solvent is removed under vacuum and the crude product is purified by FPLC ($C_{18}$, 90/10 $H_2O$/ACN to 100 % ACN) to give bispidol **9** (90 mg) with a 50 % yield.

$^1$H NMR (MeOD, 400 MHz): δ 1.69 (m, 8H, H17 + H12 + H13); 1.90 (s, 3H, H15); 2.16 (m, 1H, H11/11'); 2.33 (m, 1H, H11/11'); 3.21 (m, 2H, H14); 3.57 (s, 3H, H16/16'); 3.63 (s, 3H, H16/16'); 3.76 (m, 1H, H10); 3.91 (AB system, $\delta_A$ = 3.84 , $\delta_B$ = 3.97, $J_{AB}$ = 12.2 Hz, 2H, H6/8ax + H6/8eq); 4.14 (AB system, $\delta_A$ = 4.01 , $\delta_B$ = 4.26, $J_{AB}$ = 12.2 Hz, 2H, H6/8ax + H6/8eq); 4.49 (s, 1H, H9); 4.87 (s, 1H, H2/4); 4.92 (s, 1H, H2/4); 7.39 (m, 2H, Hb/b'); 7.60 (d, *J* = 8.0 Hz, 2H, Hd/d'); 7.83 (m, 2H, Hc/c'); 8.65 (m, 2H, Ha/a'); 8.68 (m, 2H, Ha/a').

$^{13}$C NMR (MeOD, 126 MHz): δ 22.75 (C15); 25.47 ($CH_2$-lys); 30.24 ($CH_2$-lys); 30.42 ($CH_2$-lys); 40.52 ($CH_2$-lys); 41.92 (C17); 51.40 (C6/8); 52.54 ($C^4$); 52.59 ($C^4$); 53.15 (C16/16'); 53.25 (C16/16'); 57.01 (C6/8); 67.62 (C2/4); 68.04 (C2/4); 72.12 (C10); 74.04 (C9); 125.11 (Cb/b'); 125.17 (Cb/b'); 128.81 (Cd/d'); 128.88 (Cd/d'); 138.24 (Cc/c'); 138.37 (Cc/c'); 150.35 (Ca/a'); 150.85 (Ca/a'); 157.80 ($C^4$); 157.95 ($C^4$); 170.21 ($C^4$); 170.62 ($C^4$); 173.18 ($C^4$); 173.25 ($C^4$).

Mass Spectrometry: (ESI$^+$/MS): m/z = 598.29 $[M + H]^+$, calculated for $C_{30}H_{39}N_5O_8$: 597.28.

*Compound **(I-d)**.* Lithium hydroxide (32 mg, 35 mmol, 5 eq) is dissolved in a solution of bispidol **9** (160 mg, 0.27 mmol, 1 eq) in a mixture of THF (3 ml) and $H_2O$ (7 ml). The mixture is stirred at room temperature for 16 hours. At the end, the solvent is removed under vacuum. The crude product is dissolved in a minimum of water, the pH of the solution is lowered at 2 and the compound is then purified by FPLC ($C_{18}$, 97/3 $H_2O$/ACN to 100 % ACN) to give compound **(I-d)**.0.5LiCl (110 mg) with a 69 % yield.

$^1$H NMR ($D_2O$, 600 MHz): δ 1.52 (m, 2H, H12); 1.68 (m, 2H, H13); 1.89 (s, 3H, H16); 1.93 (s, 3H, H15); 1.96 (m, 1H, H11/11'); 2.18 (m, 1H, H11/11');3.35 (AB system $\delta_A$ = 3.08, $\delta_B$ = 3.62, 2H, H6/8ax + H6/8eq); 3.52 (AB system $\delta_A$ = 3.23, $\delta_B$ = 3.82, 2H, H6/$8_{ax}$ + H6/$8_{eq}$); 3.23 (m, 2H, H14); 3.62 (m, 1H, H10); 4.26 (s, 1H, H9); 4.90 (m, 2H, H2/4); 7.42 (m, 2H, Hb); 7.55 (m, 2H, Hd); 7.84 (m, 2H, Hc); 8.59 (m, 2H, Ha).

$^{13}$C NMR ($D_2O$, 150 MHz): δ 21.85 (C15); 23.25 ($CH_2$-lys); 28.06 ($CH_2$-lys); 28.21 ($CH_2$-lys); 39.23 ($CH_2$-lys); 41.90 (C16); 50.19 ($C^4$); 50.72 ($C^4$); 52.07 (C6/8); 54.79 (C6/8) 67.84 (C2/4); 68.00 (C2/4) 71.42 (C10); 72.98 (C9); 123.86 (Cb/b'+ Cb/b'); 125.19 (Cd/d'); 126.34 (Cd/d'); 137.59 (Cc/c'); 137.64 (Cc/c'); 148.83 (Ca/a'); 149.09 (Ca/a'); 155.59 ($C^4$); 155.76 ($C^4$); 173.88 ($C^4$); 174.48 ($C^4$); 175.00 ($C^4$); 175.61 ($C^4$).

Mass Spectrometry: (ESI$^+$/MS): m/z = 570.25 $[M+ H]^+$ calculated for $C_{28}H_{35}N_5O_8$: 569.25.

Elemental Analysis: Calculated for $C_{28}H_{35}N_5O_8$.0.5(LiCl), C, 56.92, H, 5.97, N, 11.85. Found: C, 56.82, H, 6.01, N, 11.96.

Example 6: synthesis of compound (I-e)

Compound (I-e) was prepared according to the following scheme 7:

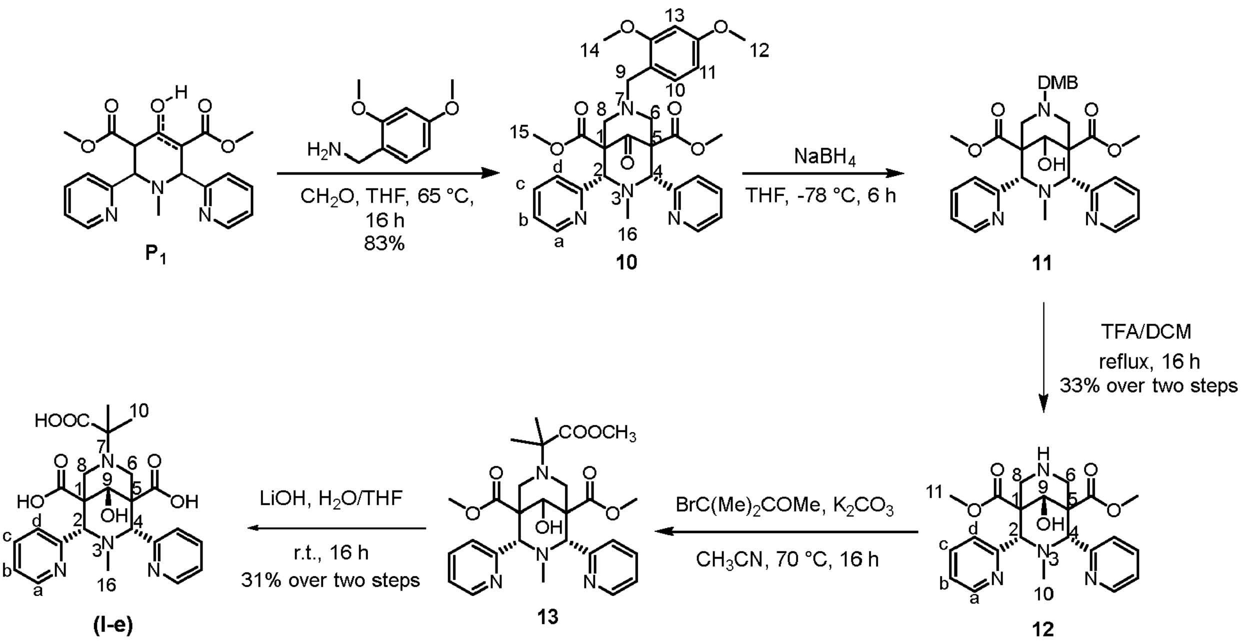


## SCHEME 7

*Compound* ***10.*** $\mathbf{P_1}$ (dimethyl-1-methyl-4-oxo-2,6-di(pyridin-2-yl)piperidine-3,5-dicarboxylate, 1750 mg, 4.56 mmol, 1 eq) is dissolved in THF (40 ml). 2,4-Dimetoxybenzylamine (910 mg, 5.45 mmol, 1.2 eq) and formaldehyde 37 % (88μl, 11 mmol, 2.4 eq) are then added successively. The mixture is heated at reflux for 16 hours and the reaction is monitored by TLC ($SiO_2$, DCM/MeOH as eluent). At the end of the reaction, solvent is removed and the solid is washed with hot MeOH yielding compound **10** as a colourless solid (2.15 g, 83 %).

$^1H$ NMR ($CDCl_3$, 400 MHz): $\delta$ 1.91 (s, 3H, H16); 2.76 (AB system, $\delta_A$ = 2.46; $\delta_B$ = 3.05 d, $J_{AB}$ = 12.0 Hz, 4H, H6/8); 3.36 (s, 2H, H9); 3.8 (s, 3H, H12/H14), 3.84 (s, 6H, H15), 3.93 (s, 3H, H12/H14); 4.65 (s, 2H, H2 + H4); 6.41 (dd, $J_1$ = 8.2 Hz, $J_2$ = 2.5 Hz, 1H, H11); 6.57 (d, $J$ = 2.5 Hz, 1H, H13); 7.02 (d, $J$ = 8.2 Hz, 1H, H10); 7.1 (m, 2H, Hb); 7.46 (td, $J_1$ = 7.8 Hz, $J_2$ = 1.9 Hz, 2H, Hc); 7.98 (d, $J$ = 7.8 Hz, 2H, Hd); 8.34 (m, 2H, Ha).

$^{13}C$ NMR ($CDCl_3$, 126 MHz): $\delta$ 43.24; 52.48; 55.56; 55.82; 59.08; 62.11; 74.04; 98.79; 103.79; 117.77; 122.84; 123.74; 133.2; 136.16; 148.90; 158.75; 159.35; 160.77; 168.68; 204.09.

Mass Spectroscopy ($ESI^+$/MS): m/z = 575.25 $[M + H]^+$, calculated for $C_{31}H_{34}N_4O_7$: 574.24.

*Compound* ***12.*** In a solution of compound **10** (6.56 g, 11,4 mmol, 1 eq) in THF at -78°C, 0.33 eq of sodium borohydride (0.28 g, 7.52 mmol, 0.66 eq in total) is added two times at 2 hours of interval and the mixture is stirred at -78 °C for 2 hours. At the end, the reaction is quenched with 40 ml of $NH_4Cl$ and the solvent is removed under vacuum. The DCM is added, salts are filtered and the solvent is removed under vacuum. The crude product is used for the next step without purification and it is dissolved in a mixture of DCM (15 ml) and TFA (10 ml). The mixture is stirred at reflux for 16 hours. At the end, the solvent is evaporated and the crude product is dissolved in 20 ml of MeOH and stirred at reflux for 2 hours. A white precipitate is obtained, which is then filtered and the solvent of the solution is removed under vacuum. Finally, the crude product is purified by FPLC ($SiO_2$, 100 % DCM to 80/20 DCM/MeOH) to give compound **12** (1.62 g) with a 33 % yield.

$^1H$ NMR (MeOD, 400 MHz): $\delta$ 1.53 (s, 3H, H10); 3.60 (s, 6H, H11); 3.96 (AB system, $\delta_A$ = 3.60, $\delta_B$ = 4.31, $J_{AB}$ = 13.4 Hz, 4H, H6/$8_{ax}$ + H6/$8_{eq}$); 4.53 (s, 1H, H9); 4.75 (s, 2H, H2/4); 7.35(m, 2H, Hb/b'); 7.54 (d, J = 7.6 Hz, 2H, Hd/d'); 7.97 (td, $J_1$ = 7.6 Hz, $J_2$ = 1.9 Hz, 2H, Hc/c'); 8.64 (m, 2H, Ha/a').

Compound **(1-e).** Potassium carbonate (106.4 mg, 0.77 mmol, 1.1 eq) is dissolved in a solution of compound **12** (300 mg, 0.70 mmol, 1 eq) in 30 ml of anhydrous ACN under argon. Then, methyl 2-bromo-2-methylpropionoate (100 μl, 0.77 mmol, 1.1 eq) is added and the mixture is heated at 80 °C for 16 hours. At the end, $K_2CO_3$ is filtered and solvent is removed under vacuum. The product is used for the next step without purification. The crude product is dissolved in a mixture of $H_2O$ (20 ml) and THF (10 ml) and lithium hydroxide (83.82 mg, 3.5 mmol, 5 eq) is added. The solution is stirred for 16 hours at room temperature. At the end, the solvent is evaporated and the crude product is dissolved in a minimum of $H_2O$ and the pH is lowered to 2 with aqueous HCl. The solution is then purified by FPLC ($C_{18}$, 97/3 $H_2O$/MeOH to 50/50 $H_2O$/MeOH) to give compound **(1-e)**.2HCl.0.5$H_2O$ (125 mg) with a 31 % yield.

$^1H$ NMR (MeOD, 400 MHz) : $\delta$ 1.13 (s, 6H, H11); 2.08 (s, 3H, H10); 2.50 (AB system, $\delta_A$ = 2.39 , $\delta_B$ = 2.60, $J_{AB}$ = 12.8 Hz, 4H, H6/8ax + H6/8eq); 4.11 (s, 1H, H9); 5.03 (s, 2H, H2 + H4); 7.66 (m, 4H, Hb + Hd); 8.06 (td, $J_1$ = 7.9

Hz, $J_2$ = 1.3 Hz, 2H, Hc); 8.73 (m, 2H, Ha).

$^{13}C$ NMR (MeOD, 126 MHz): $\delta$23.15; 46.61; 51.41; 53.23; 53.79; 67.70; 68.74; 75.18; 128.14; 128.93; 142.13; 150.87; 157.81; 178.60; 186.65.

Mass Spectrometry: (ESI$^+$/MS): m/z = 485.19 [M + H]$^+$, calculated for $C_{24}H_{28}N_4O_7$: 484.20.

Elemental Analysis: Calculated for $C_{24}H_{28}N_4O_7.2HCl.0.5H_2O$, C, 50.89, H, 5.52, N, 9.89. Found: C, 50.77, H, 5.71, N, 10.33.

Example 7: synthesis of compound (I-f)

Compound (I-f) was prepared according to the following scheme 8:

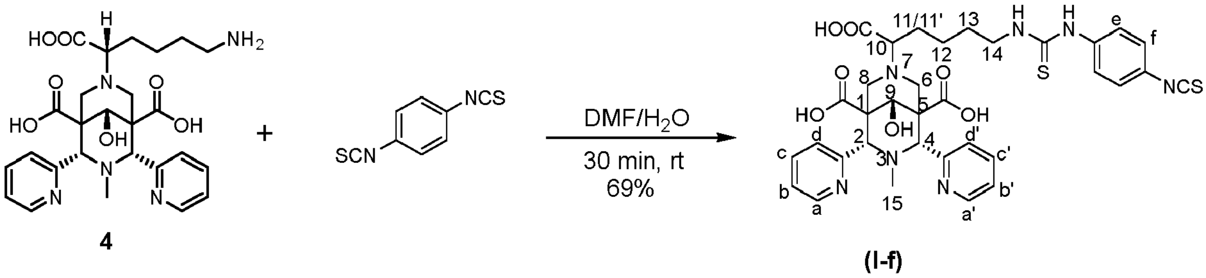


**4**

**(I-f)**

**SCHEME 8**

*Compound **(I-f)**.* Compound **4** (70 mg, 0.13 mmol, 1 eq) is dissolved in $H_2O$ (2.5 ml) and DMF (2 ml). p-phenylene diisothiocyanate (PDITC) (291 mg, 1.33 mmol, 10 eq) is then added to the solution and the resulting mixture is stirred during 30 min. At the end, the excess of PDITC is filtered. $H_2O$ is added to the solution and the observed precipitate is filtered. The solvent is then removed under vacuum at 35 °C to finally give compound **(I-f)**.3NaOH.$H_2O$ (77 mg) with a 69 % yield.

$^1H$ NMR ($D_2O$, 400 MHz): $\delta$1.62 (m, 2H, H12); 1.79 (m, 2H, H13); 1.88 (s, 3H, H15); 2.00 (m, 1H, H11/H11'); 2.20 (m, 1H, H11/H11'); 3.17 (AB system, $\delta_A$ = 3.13, $\delta_B$ = 3.21, $J_{AB}$ = 12.3 Hz, 2H, H6/H8); 3.56 (m, 4H, H10 + H14 + H8/H6); 3.81 (m, 1H, H6/H8); 4.28 (s, 1H, H9); 4.91 (m, 2H, H2 + H4); 7.23 (m, 2H, He/Hf), 7.31 (m, 2H, He/Hf); 7.43 (m, 2H, Hd/Hd'); 7.59 (m, 2H, Hb/Hb'); 7.86 (m, 2H, Hc/Hc'); 8.64 (m, 2H, Ha/Ha').

Mass Spectrometry: (ESI$^+$/MS): m/z = 720.23 [M + H$^+$], calculated for $C_{34}H_{37}N_7O_7S_2$, 729.22.

Elemental Analysis: Calculated for $C_{36}H_{41}N_7O_7S_2.3NaOH.H_2O$, C, 47.60, H, 4.93, N, 11.43, S, 7.48. Found: C, 47.89, H, 4.92, N, 11.61, S, 7.24.

Example 8: synthesis of compound (I-g)

Compound (I-g) was prepared according to the following scheme 9:

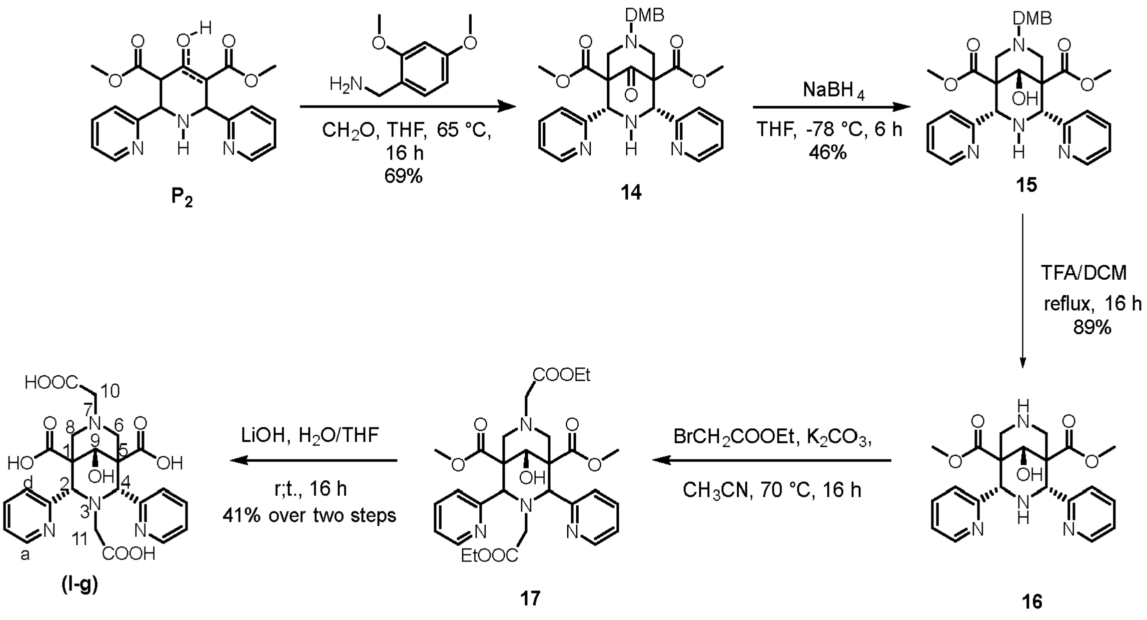


## SCHEME 9

$\mathbf{P_2}$ is a well-known starting material to lead to bispidone and bispidine compounds.

*Compound **14**.* Piperidone $\mathbf{P_2}$ (20.0 g, 54.2 mmol, 1.0 eq) was dissolved in 200 ml tetrahydrofuran and heated to 50°C. 2,4-dimethoxybenzylamine (49.8 ml, 65.0 mmol, 1.2 eq) and formaldehyde (9.76 ml, 37 % in MeOH / $H_2O$, 130 mmol, 2.4 eq) were added successively via a dropping funnel. The reaction mixture was heated under reflux overnight. After cooling to room temperature the solvent was removed under reduced pressure and the remaining solid was washed with hot MeOH yielding compound **14** as a colorless solid (20.8 g, 37.0 mmol, 69 %).

$^1H$ NMR (600.13 MHz, 25 °C, $CDCl_3$): $\delta$ = 2.92 (d, $^2J_{H,H}$ = 11.5 Hz, 2 H, N7$CH_{2ax,eq}$), 3.28 (bs, 2 H, N7$CH_2$), 3.61-3.62 (m, 5 H, N7$CH_{2ax,eq}$, o-$OCH_3$), 3.74 (s, 6 H, $COOCH_3$), 3.79 (s, 3 H, p-$OCH_3$), 5.10 (bd, $^3J_{H,H}$ = 11.6 Hz, 2 H, N3CH), 5.22 (bt, $^3J_{H,H}$ = 11.6 Hz, 1 H, N3H), 6.27 (bd, $^3J_{H,H}$ = 7.3 Hz, 1 H, Hph), 6.30 (d, $^4J_{H,H}$ = 1.9 Hz, 1 H, Hph), 6.96 (d, $^3J_{H,H}$ = 7.3 Hz, 1 H, Hph), 7.13 (bt, $^3J_{H,H}$ = 4.2 Hz, 2 H, Hpy), 7.53 (d, $^3J_{H,H}$ = 7.0 Hz, 2 H, Hpy), 7.61 (bt, $^3J_{H,H}$ = 7.0 Hz, 2 H, Hpy), 8.37 (bd, $^3J_{H,H}$ = 4.2 Hz, 2 H, Hpy) ppm.

$^{13}C$ NMR (150.92 MHz, 25 °C, $CDCl_3$): $\delta$ = 52.1, 54.9, 55.4, 55.4, 57.6, 61.3, 66.5, 98.3, 103.3, 118.0, 122.3, 122.8, 131.7, 136.2, 148.5, 157.5, 158.9, 160.2, 169.9, 205.5 ppm.

*Compound **15**.* Compound **14** (6.00 g, 10.7 mmol, 1.0 eq) was dissolved in THF and cooled to -78 °C. Sodium borohydride (203 mg, 5.35 mmol, 0.5 eq) was added in two times at 2 hours of interval and the solution was stirred overnight at -78°C for 2 hours. At the end, the reaction is quenched with $NH_4Cl$ and the solvent is removed under vacuum. The DCM is added, salts are filtered and the solvent is removed under vacuum. The crude product is used for the next step without purification (46 % yield).

*Compound **16**.* Compound **15** is dissolved in a mixture of DCM (15 ml) and TFA (10 ml). The mixture is stirred at reflux for 16 hours. At the end, the solvent is evaporated and the crude product is dissolved in 20 ml of MeOH and stirred at reflux for 2 hours. A white precipitate is obtained, which is then filtered and the solvent of the solution is removed under vacuum. The TFA salt of compound **16** was obtained as colorless crystals (89 % yield) from the concentrated filtrate.

$^1H$ NMR (600.13 MHz, 25 °C, MeOH-$d^4$): $\delta$ = 3.48 (dd, $^2J_{H,H}$ = 13.2 Hz, $^3J_{H,H}$ = 1.2 Hz, 2 H, N7$CH_{2ax,eq}$), 3.62 (bd, $^2J_{H,H}$ = 13.2 Hz, 2 H, N7$CH_{2ax,eq}$), 3.69 (s, 6 H, $COOCH_3$), 4.76 (d, $^3J_{H,H}$ = 1.4 Hz, 2 H, N3CH), 4.89 (s, 1 H, CHOH), 7.40 (ddd, $^3J_{H,H}$ = 7.7 Hz, $^3J_{H,H}$ = 4.8 Hz, $^4J_{H,H}$ = 1.2 Hz, 2 H, Hpy), 7.49 (d, $^3J_{H,H}$ = 7.7 Hz, 2 H, Hpy), 7.85 (td, $^3J_{H,H}$ = 7.7 Hz, $^4J_{H,H}$ = 1.8 Hz, 2 H, Hpy), 8.63 (ddd, $^3J_{H,H}$ = 4.8 Hz, $^3J_{H,H}$ = 1.8 Hz, $^4J_{H,H}$ = 0.9 Hz, 2 H, Hpy) ppm.

$^{13}C$ NMR (150.92 MHz, 25 °C, MeOH-$d^4$): $\delta$ = 41.4, 51.9, 53.2, 67.6, 73.3, 124.7, 125.3, 139.0, 150.8, 158.2, 171.6 ppm.

HR-ESI MS (pos, MeOH): [5+H]+ calcd 413.18195, obsd 413.18221.

Elemental analysis (report no. 39141): [5.H(TFA)] calcd C, 52.47; H, 4.79; N, 10.64 %; obsd C, 52.72; H, 4.93; N, 10.57 %.

*Compound **(I-g)**.* Potassium carbonate (212 mg, 1.53 mmol, 2.4 eq) is dissolved in a solution of compound **16** (263 mg, 0.64 mmol, 1 eq) in 50 ml of anhydrous ACN under argon. Then, ethyl bromoacetate (182 $\mu$l, 1.6 mmol, 2.5 eq) is added and the mixture is heated at 80 °C for 16 hours. At the end, $K_2CO_3$ is filtered and the solvent is removed under vacuum. The product is used for the next step without purification. The crude product is dissolved in a mixture of

$H_2O$ (10 ml) and THF (5 ml) and lithium hydroxide (123 mg, 5.12 mmol, 8 eq). The solution is stirred for 16 hours at room temperature. At the end, the solvent is evaporated and the crude product is purified by FPLC ($C_{18}$, , 97/3 $H_2O$/MeOH to 50/50 $H_2O$/MeOH) to give compound **(I-g).** 2 LiOH.$H_2O$ (150 mg) with a 41 % yield.

$^1H$ NMR (MeOD, 400 MHz) : $\delta$ 2.82 (s, 2H, H10/H11); 3.68 (AB system, $\delta_A$ = 3.60 , $\delta_B$ = 3.75, 4H, H6/$8_{ax}$ + H6/$8_{eq}$); 3.75 (s, 2H, H10/H11); 4.42 (s, 1H, H9); 5.75 (s, 2H, H2/H4); 7.36 (m, 2H, Hb); 7.65 (m, 2H, Hd); 7.80 (m, 2H, Hc); 8.67 (m, 2H, Ha).

$^{13}C$ NMR ($D_2O$, 150 MHz): $\delta$50.83; 56.27; 59.10; 64.07; 72.81; 124.66; 127.54; 140.64; 146.92; 155.00; 170.92; 174.71; 176.56.

Mass Spectrometry: (ESI$^-$/MS): m/z = 499.14 [M - H]$^-$; 505.15 [M - 2H + Li]$^-$, calculated for $C_{23}H_{24}N_4O_9$: 500.15

Elemental Analysis: Calculated for $C_{23}H_{24}N_4O_9$.2(LiOH).$H_2O$, C, 48.78, H 4.98, N, 9.89. Found: C,48.78, H, 5.02, N, 10.16.

Example 9: synthesis of a complex of manganese (II) with bispidine-based metal chelating ligands (I-a)

A complex (I-A) of manganese (II) with chelating bispidine-based ligands (I-a) of example 2 was prepared as follows:

Equimolar quantities of ligand and $MnCl_2$ were dissolved in milli-Q water, the pH was adjusted to 7.4 either with a buffered solution or by adding KOH or HCl to the solution and the samples were heated at 60 °C for 30 min. The absence of free $Mn^{2+}$ was checked by the Xylenol orange test.

The properties of complex (I-A) in terms of relaxivity and stability were compared to the properties of two other promising Mn-based constrat agents, namely MnPC2A-EA and MnPyC3A, the latest compound being patented and already tested in clinical trials in baboons.

Table 1 below shows the properties of complex (I-A) and for comparison of MnPC2A-EA and MnPyC3A .

**TABLE 1**

| Compound | Longitudinal relaxivity in water ($r_1$; $mM^{-1}/s$) at 25°C, pH = 7.4, and 60 MHz | Half-life in excess of $Zn^{2+}$(25 eq, pH =6, 37°C) ($t_{1/2}$) |
|---|---|---|
| **(I-A)** | 4.89 | NA |
| **(I-D)** | 4.03 | 160 days |
| **MnPC2A-EA** | 3.52 | 54.4 hours |
| **MnPyC3A1** | 3.3 | 0.285 hours |
| NA : not available | | |

The relaxivity at 60MHz is measured with a device commercialized under the brand name WP80 NMR by the firm Brucker according to the following protocol. A milimolar aqueous solution of complex was prepared with Milli-Q water ($\rho$ < 18M$\Omega$). The concentration of $Mn^{2+}$-containing samples was checked by ICP-OES and/or NMR by using the bulk magnetic susceptibility. The absence of free $Mn^{2+}$ was checked by the Xylenol orange test. For the relaxivity measurement, the temperature was monitored by a VTC91 temperature control unit and maintained by a gas flow. The temperature was determined by previous calibration with a Pt resistance temperature probe.

The kinetic inertness of the maganese(II) complexes and in particular their half-life was assessed at 37°C and in 0.1 M KCl, via transmetallation studies of $Mn^{2+}$ complex (1 mM) with $Zn^{2+}$ (as $ZnCl^2$) at pH 6.06 (0.030 M MES buffer, in the presence of 10- and 50-fold excess of the exchanging $Zn^{2+}$), in the presence of 50-fold excess of $Zn^{2+}$. The excess of the exchanging metal ion guarantees the pseudo-first order conditions. For the duration of the experiments (up to 3 months), the samples were stored in a thermostat at 37°C between the relaxivity measurements. The pH was controlled for each sample at the end of the kinetic measurements to confirm that it remained stable during the experiment. The reactions were monitored by measuring the water proton relaxation rates at 60 MHz on a relaxometer commercialized under the brand name Minispec by the firm a Bruker. The analysis of the experimental data was performed using Visualiseur/Optimiseur running on a MATLAB 8.3.0 (R2014a) platform according to equations (1) and (2) :

$$-\frac{d[MnL]_t}{dt} = k_{obs}[\mathrm{MnL}]_t \qquad Equation\,(1)$$

$$t_{1/2} = \ln(2)/k_{obs}$$

were $[MnL]_t$ stands for the total concentration of MnL complex, $k_{obs}$ stands for the pseudo-first order constant and $t_{1/2}$ is the half-life of the MnL complex in the given conditions.

Based on these results in Table 1, it can be concluded that the ligands of the present invention lead to complexes having improved relaxivity while guaranteeing good kinetic inertness. Additionally, the complexes obtained do not involve gadolinium metal and have therefore a reduced toxicity.

## Claims

**1.** A bispidine-based metal chelating ligand responding to the following formula (I):

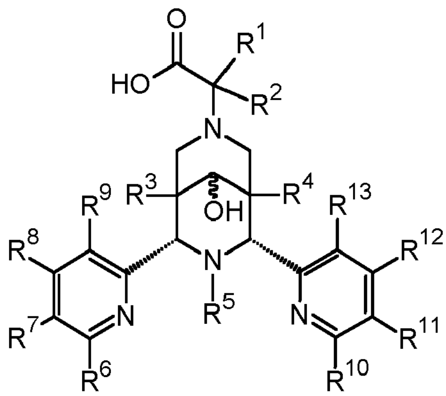

**(I)**

in which:

* $R^1$ represents an hydrogen atom or a $C_1$-$C_5$ alkyl group,
* $R^2$ represents an hydrogen atom, a $C_1$-$C_5$ alkyl group, a group responding to formula (II): $-(CH_2)_n-NH_2$ (II), where $3 \leq n \leq 18$, or a group responding to the following formula (III):

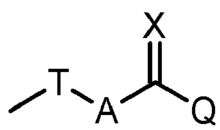

**(III)**

where:

* T represents a $C_1$-$C_{17}$ alkylene group, a $C_1$-$C_{17}$ alkenylene group, or a $C_1$-$C_{17}$ alkynylene group,
* A represents -CH2- or -NH-,
* X represents an oxygen atom, a sulfur atom, or a NH group, and
* Q represents:

- an alkyl group optionally substituted with a functional group,
- a $NHR^{14}$ group where $R^{14}$ represents an hydrogen atom, or an aryl group optionally substituted with a functional group,
- a polyethylene glycol group responding to formula (IV): $-CH_2-(CH_2-O-CH_2)_q-CH_2OH$ (IV), where $1 \leq q \leq 24$, or
- an OH group,

* $R^3$ and $R^4$, which may be identical or different, represent a $CH_2OH$ group, a $CO_2H$ group, or a $CONHR^{15}$

group, where $R^{15}$ represents:

- an alkyl group optionally substituted with a functional group,
- an aryl group optionally substituted with a functional group, or
- a polyethylene glycol group responding to formula (V): $-CH_2-(CH_2-O-CH_2)_r-CH_2OH$ (V), where $1 \leq r \leq 24$,

* $R^5$ represents an hydrogen atom, an alkyl group, a group of formula (VI): $-(CH_2)_m-CO_2H$ (VI) where $1 \leq m \leq 5$, a polyethylene glycol group responding to formula (VII): $-CH_2-(CH_2-O-CH_2)_s-CH_2OH$ (VII), where $1 \leq s \leq 18$, or a group of formula (III) as defined above,
* $R^6$, $R^7$, $R^8$, $R^9$, $R^{10}$, $R^{11}$, $R^{12}$, and $R^{13}$, which may be identical or different, represent a hydrogen atom, an OH group, an ether group $OR^{16}$ where $R^{16}$ is an alkyl group, a $CO_2H$ group, or a $CONHR^{17}$ group, where $R^{17}$ represents an alkyl group optionally substituted with a functional group,

with the proviso that:

- when $R^2$ represents a group responding to formula (II): $-(CH_2)_n-NH_2$ (II), and $R^5$ represents an alkyl group, then $R^3$ and $R^4$ are different from $CO_2H$ groups,
- when $R^5$ represents an alkyl group, then $R^1$ and $R^2$ are different from hydrogen atoms, and
- when $R^2$ represents a group of said formula (III), where T represents a $C_1$-$C_{17}$ alkylene group, A represents -NH-, X represents an oxygen atom, and Q represents a $C_1$-$C_5$ alkyl group substituted with a functional group, then $R^3$ and $R^4$ are different from $CO_2H$ groups.

**2.** The ligand according to claim 1, wherein T represents a $C_1$-$C_{17}$ alkylene group.

**3.** The ligand according to claim 1 or claim 2, wherein the alkyl group optionally substituted with a functional group as a Q group is a linear $C_5$-$C_{20}$ alkyl group optionally substituted with a functional group.

**4.** The ligand according to any one of the preceding claims, wherein the functional group is selected from the following groups: amide, sulfonate, sulfate, quaternaty ammonium, hydroxyl, phosphonate, succinimidyl ester, sulfosuccinimidyl ester, isothiocyanate, isocyanate, iodoacetamide, maleimide, sulfonyl halide, acid halide, carbodiimide, biotin, azido, alkyne, and a -C(O)-Z group, where Z is selected from an -OH, -N-glycine, -N-lysine, $-Y-L-NH_2$, -Y-L-COOH and -Y-L-SH group, where Y is selected from N and O atoms, and L is selected from a $C_1$-$C_{17}$ alkylene group, a $C_1$-$C_{17}$ alkenylene group, a $C_1$-$C_{17}$ alkynylene group, and a phenylene group.

**5.** The ligand according to any one of the preceding claims, wherein the $NHR^{14}$ group as the Q group is such that $R^{14}$ represents a $C_5$-$C_{20}$ aryl group optionally substituted with a functional group.

**6.** The ligand according to any one of the preceding claims, wherein $R^2$ represents a group responding to said formula (II) or (III).

**7.** The ligand according to any one of the preceding claims, wherein the group of formula (III) is such that:

- A represents -NH-, X represents an oxygen atom, and Q represents an alkyl group optionally substituted with a functional group;
- A represents -NH-, X represents a sulfur atom, and Q represents a $NHR^{14}$ group where $R^{14}$ represents an aryl group optionally substituted with a functional group; or
- A represents $-CH_2-$, X represents an oxygen atom, and Q represents an OH group.

**8.** The ligand according to any one of the preceding claims, wherein $R^3$ and $R^4$, which may be identical or different, represent a $CH_2OH$ group or a $CO_2H$ group.

**9.** The ligand according to any one of the preceding claims, wherein $R^3$ and $R^4$ are identical.

**10.** The ligand according to any one of the preceding claims, wherein $R^5$ represents an alkyl group or a group of formula (VI): $-(CH_2)_m-CO_2H$ (VI) where $1 \leq m \leq 5$.

**11.** The ligand according to any one of the preceding claims, wherein the alkyl group as $R^5$ is a linear $C_1$-$C_5$ alkyl group.

**12.** The ligand according to any one of the preceding claims, wherein $R^6$, $R^7$, $R^8$, $R^9$, $R^{10}$, $R^{11}$, $R^{12}$, and $R^{13}$ groups

represent hydrogen atoms or at least $R^7$, $R^9$, $R^{11}$, and $R^{13}$ represent hydrogen atoms.

**13.** The ligand according to any one of the preceding claims, wherein it is selected from the following formulae (I-a) to (I-g):

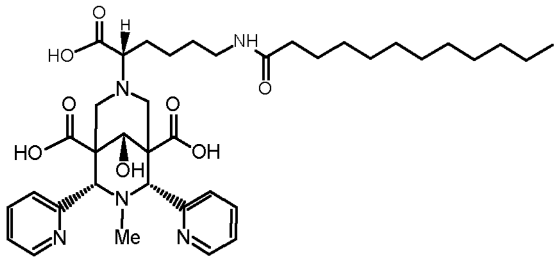

**(I-a)**

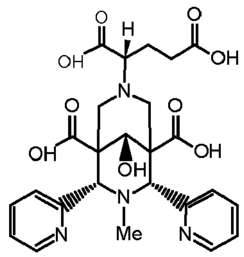

**(I-b)**

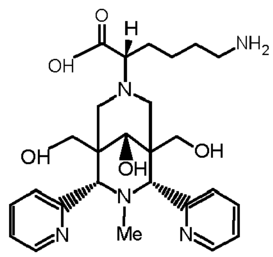

**(I-c)**

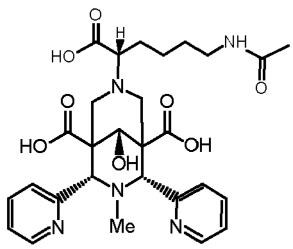

**(I-d)**

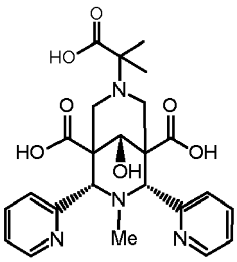

(I-e)

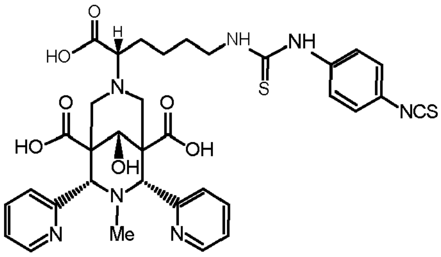

(I-f)

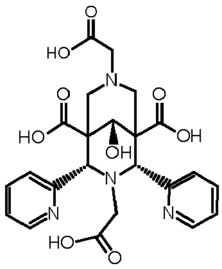

(I-g) .

**14.** A complex of a metal M, wherein said complex comprises a metal ion of said metal M complexed with a bispidine-based metal chelating ligand (I) as defined in any one of the preceding claims, said metal M being selected from copper, manganese, gallium, cobalt, zinc, nickel, and iron.

**15.** Use of a bispidine-based metal chelating ligand (I) as defined in any one of claims 1 to 13, or of a complex as defined in claim 14, in the field of medical imaging or therapy, and more specifically as MRI contrast agents and/or nuclear imaging agents for PET or SPECT.

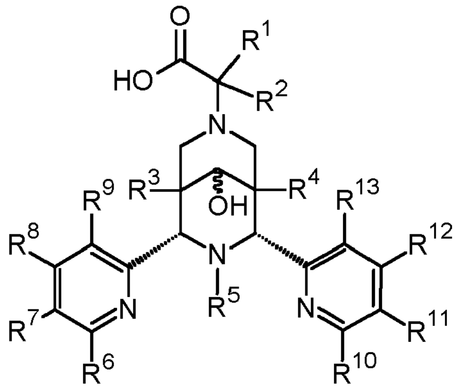

(I)

FIG. 1

Europäisches Patentamt
European Patent Office
Office européen des brevets

# EUROPEAN SEARCH REPORT

Application Number
EP 21 30 6966

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DAOUDA NDIAYE ET AL: "Unprecedented Kinetic Inertness for a Mn2+-Bispidine Chelate: A Novel Structural Entry for Mn2+-Based Imaging Agents", ANGEWANDTE CHEMIE, WILEY - V C H VERLAG GMBH & CO. KGAA, DE, vol. 132, no. 29, 18 May 2020 (2020-05-18), pages 12056-12061, XP071381370, ISSN: 0044-8249, DOI: 10.1002/ANGE.202003685 * H3L1, Scheme 1 Experimental * ----- | 1-15 | INV. C07D471/08 C07B59/00 A61K51/04 |
| Y | ROUX AMANDINE ET AL: "Bifunctional bispidine derivatives for copper-64 labelling and positron emission tomography", ORGANIC & BIOMOLECULAR CHEMISTRY, vol. 15, no. 6, 1 January 2017 (2017-01-01), pages 1475-1483, XP055922523, ISSN: 1477-0520, DOI: 10.1039/C6OB02712A * experimental; figure 1 * ----- | 1-15 | TECHNICAL FIELDS SEARCHED (IPC) C07D C07B |
| Y | NONAT ALINE M. ET AL: "2,4-Substituted bispidines as rigid hosts for versatile applications: from [kappa]-opioid receptor to metal coordination", DALTON TRANSACTIONS, vol. 48, no. 44, 1 January 2019 (2019-01-01), pages 16476-16492, XP055922360, Cambridge ISSN: 1477-9226, DOI: 10.1039/C9DT03480C * page 16489 * ----- -/-- | 1-15 | |

The present search report has been drawn up for all claims

1

| Place of search | Date of completion of the search | Examiner |
|---|---|---|
| Munich | 24 May 2022 | Österle, Carmen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 30 6966

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ROUX AMANDINE ET AL: "Kinetically Inert Bispidol-Based Cu(II) Chelate for Potential Application to 64/67 Cu Nuclear Medicine and Diagnosis", INORGANIC CHEMISTRY, vol. 54, no. 9, 4 May 2015 (2015-05-04), pages 4431-4444, XP055922532, Easton , US ISSN: 0020-1669, DOI: 10.1021/acs.inorgchem.5b00207 * Chart 1 * ----- | 1-15 | |

TECHNICAL FIELDS SEARCHED (IPC)

The present search report has been drawn up for all claims

1

| Place of search | Date of completion of the search | Examiner |
|---|---|---|
| Munich | 24 May 2022 | Österle, Carmen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2020157099 A1